# EUROPEAN PATENT SPECIFICATION

(11) **EP 1 502 948 B1**
(45) Date of publication and mention of the grant of the patent: **17.12.2008**
(21) Application number: 04077888.8
(22) Date of filing: 17.05.2001
(51) Int. Cl.: C12N 1/20, C12N 9/06, C12N 15/53, C02F 3/34, C12Q 1/68

(54) **Ammonia-oxidizing bacteria**
Ammoniak oxidierende Bakterien
Bactéries oxydant l'ammoniac

(30) Priority: 19.05.2000 US 573684
(43) Date of publication of application: 02.02.2005
(62) Divisional of application: 01937587.2
(73) Proprietor: Aquaria Inc., Moorpark, CA 93021 (US)
(72) Inventor: Hovanec, Timothy A., Moorpark, CA 93021 (US); Burrel, Paul C., Orange, New South Wales 2800 (AU)
(74) Representative: Martin, David John

(56) References cited:
- DATABASE EMBL 5 September 1994 (1994-09-05), TESKE ET AL: "Nitrosolobus multiformis (ATCC 25196) 16S ribosomal RNA (16S rRNA) gene" XP002309485 retrieved from EBI accession no. EMBL:NM16SR Database accession no. L35509
- DATABASE EMBL 29 November 1994 (1994-11-29), TESKE ET AL: "Nitrosospira briensis (strain C-128) 16S ribosomal RNA (16S rRNA) gene" XP002309486 retrieved from EBI accession no. EMBL:NM16SR Database accession no. L35505
- HOVANEC ET AL: "Comparative analysis of nitrifying bacteria associated with freshwater and marine aquaria" APPLIED AND ENVIRONMENTAL MICROBIOLOGY, vol. 62, 1996, pages 2888-2896, XP002267477

## Description

### BACKGROUND OF THE INVENTION

### 1. Field of Invention

The invention relates generally to ammonia oxidizers, and specifically to a new type of bacterium capable of oxidizing ammonia to nitrite, and being characterized by SEQ ID No : 3.

### 2. Background Information

Ammonia is the principal nitrogenous waste product of teleosts and many invertebrates in both freshwater and seawater. The ammonia results from the deamination or transamination of proteins the organism receives via its diet. However, high ammonia concentrations can be toxic to many of these same aquatic organisms. In natural systems, such as lakes, rivers and oceans, the concentration of ammonia rarely reaches deleterious levels because the density of fish (and other organisms) per mass of water is low.

However, in man-made aquatic systems such as aquaculture rearing pens, tanks, raceways and ponds plus aquaria, both public and private, ammonia can reach toxic concentrations, sometimes very quickly. One reason for this is that in the above-named systems the fish density can be very large in relation to the small amount of water. Another reason is that in many of these systems the water is not continually changed; rather it recirculates through the system with only periodic partial water changes.

Therefore, most aquaculture systems and aquaria use filtration, in one form or another, to maintain a degree of water quality that is suitable for the maintenance and growth of aquatic organisms. A major component of any such filtration unit is the biological filter. The biological filter gets its name from the fact that it acts as a substrate or site for the growth of bacteria which have the capability to convert, by way of oxidation, ammonia to another compound - nitrite. High concentrations of nitrite can also be toxic but there are other species of bacteria which grow on the biological filter and oxidize the nitrite to nitrate. Nitrate is considered non-toxic to aquatic organisms except in extreme cases of very high concentrations.

There are other situations or applications which use biological filters. These include sewage treatment facilities, wastewater treatment facilities and drinking water filtration plants. While each will have its own particular reason for using a biological filter, the goal is the same: the conversion of inorganic nitrogen compounds to less harmful substances. Biological filtration is necessary for many facilities to meet the National Recommended Water Quality Criteria as set by the Environmental Protection Agency (EPA) of the United States of America.

The oxidation of ammonia to nitrite is a bacterially-mediated process. Specifically, it is a two step oxidation process involving the conversion of ammonia to nitrite according to the following equations:

NH₃ + O₂ + H₂O + 2e⁻ ----- > NH₂OH + H₂O (1)

NH₂OH + H₂O ----> NO₂⁻ + 5H⁺ + 4e⁻ (2)

The most commonly studied ammonia oxidizing bacteria (AOB) is *Nitrosomonas europaea.* It was originally isolated from soils and is purported to the active AOB in aquaculture facilities (Wheaton, F. W. 1977. Aquacultural Engineering. John Wiley & Sons, Inc. New York. These references, and all other references cited herein are hereby incorporated by reference), wastewater treatment facilities (Painter, H.A. 1986. Nitrification in the treatment of sewage and waste-waters. In Nitrification J. I. Prosser ed. IRL Press. Oxford.) and in aquaria (Spotte, S. 1979. Seawater Aquariums - The Captive Environment. Wiley-Interscience. New York).

However, recent research conducted with modem molecular methods which use the uniqueness of the DNA sequence of an organism (or group of organisms) has shown that *Nitrosomonas europaea* and its close relatives were below detection limits in freshwater aquaria environments (Hovanec, T.A. and E. F. DeLong. 1996. Comparative analysis of nitrifying bacteria associated with freshwater and marine aquaria. Appl. Environ. Microbiol. 62:2888-2896.). Other research has demonstrated that *N. europaea* is not the dominant AOB in wastewater treatment facilities (Juretschko, S. et. al. 1998. Combined molecular and conventional analyses of nitrifying bacterium diversity in activated sludge: Nitrosococcus mobilis and Nitrospira-like bacteria as dominant populations. Appl. Environ. Microbiol. 64:3042-3051).

Bacteria, i.a. Nitrosospina briensis and Nitrosolobus multiformis, with sequences similar to SEQ ID NO : 3 are disclosed by Teske et al., J. Bact 176: 6623-6630, 1994.

Therefore, it is likely that there are novel, as yet, unidentified ammonia-oxidizing bacteria in these types of environments which are responsible for ammonia oxidation.

### SUMMARY OF THE INVENTION

It is the object of the present invention to provide isolated bacteria or bacterial strains capable of oxidizing ammonia to nitrite and compositions comprising such strains and methods using such strains. In a particularly preferred embodiment, the 16S rDNA of the bacteria or bacterial strains have the nucleotide sequence of SEQ ID NO:3, or a variant thereof which is at least 99% similar to SEQ ID NO:3

The invention is also intended to include nucleic acid sequences and bacteria with sequences which are at least 99% similar to , SEQ ID NO:3. For the purposes of this application "99% similar" is meant that single base substitutions may occur of up to 1% of the bases.

### BRIEF DESCRIPTION OF THE DRAWINGS

Figure 1. Phylogenetic relationships of the three bacterial strains and one substrain inferred from comparative analysis of 16S rDNA sequences. The tree is based on neighbor-joining distance analysis of sequences containing a minimum of 1430 nucleotides.
Figure 2. Denaturing gradient gel electrophoresis (DGGE) of biomasses from selected cultures and ammonia-oxidizing bacteria described herein.
Figure 3. Denaturing gradient gel electrophoresis (DGGE) demonstrating the uniqueness of the bacterial strains reported herein. There are two replicates of each bacterial type reported herein along extracts from three pure cultures of ammonia-oxidizing bacteria.
Figure 4 (A-D'). Mean ammonia and nitrite trends for the Bacterial Additives VI test.
Figure 5 (A-D'). Mean ammonia and nitrite trends for the Bacterial Additives VII test.

### DETAILED DESCRIPTION OF THE INVENTION

The present invention is based upon the discovery of novel bacterial strains which are responsible for ammonia oxidation in freshwater aquaria.

According to the first aspect of the invention there is provided an isolated bacterial strain that oxidizes ammonia to nitrite, said strain comprising the sequence set forth in SEQ ID No: 3.

According to a second aspect of the invention there is provided a biologically pure culture of a bacterial strain that oxidizes ammonia to nitrate, wherein the 16S rDNA of the bacterial strain comprises the sequence set forth in SEQ ID No: 3.

According to a third aspect of the invention there is provided a composition comprising an isolated bacterial strain that oxidizes ammonia to nitrite, wherein said bacterial strain comprises the sequence set forth in SEQ ID No: 3.

According to a fourth aspect of the invention there is provided an isolated nucleotide sequence comprising the sequence set forth in SEQ ID No: 3.

According to a fifth aspect of the invention there is provided a method of alleviating or preventing the accumulation of ammonia in an aquarium comprising a step of placing into the aquarium a sufficient amount of a bacterial strain capable of oxidizing ammonia to nitrite to alleviate the accumulation of ammonia in the aquarium, wherein said bacterial strain comprises the sequence set forth in SEQ ID No: 3 or a variant thereof which is at least 99% similar.

The present invention provides an isolated bacterial strain or a biologically pure culture of a bacterial strain capable of oxidizing ammonia to nitrite, wherein the 16S rDNA of the bacterial strain includes the nucleotide sequence of SEQ ID No: 3 as shown in table 3.

**Table 1. The sequence for the AOB Type A ammonia-oxidizing bacterium. Represented by R7clone140. SEQ ID NO:1.**

| |
|---|
| ATTGAACGCTGGCGGCATGCTTTACACATGCAAGTCGAACGGCAGCACGGAT GCTTGCATCTGGTGGCGAGTGGCGGACGGGTGAGTAATGCATCGGAACGTAT CCAGAAGAGGGGGTAACGCATCGAAAGATGTGCTAATACCGCATATACTC TAAGGAGGAAAGCAGGGGATCGAAAGACCTTGCGCTTTTGGAGCGGCCGATG TCTGATTAGCTAGTTGGTGGGGTAAAGGCCTACCAAGGCGACGATCAGTAGT TGGTCTGAGAGGACGACCAGCCACACTGGGACTGAGACACGGCCCAGACTCC TACGGGAGGCAGCAGTGGGGAATTTTGGACAATGGGCGCAAGCCTGATCCAG CAATGCCGCGTGAGTGAAGAAGGCCTTCGGGTTGTAAAGCTCTTTCAGTCGA GAAGAAAAGGTTACGGTAAATAATCGTGACTCATGACGGTATCGACAGAAG AAGCACCGGCTAACTACGTGCCAGCAGCCGCGGTAATACGTAGGGTGCAAGC GTTAATCGGAATTACTGGGCGTAAAGGGTGCGCAGGCGGCTTTGTAAGTCAG ATGTGAAATCCCCGGGCTTAACCTGGGAATTGCGTTTGAAACTACAAGGCTA GAGTGTGGCAGAGGGAGGTGGAATTCCATGTGTAGCAGTGAAATGCGTAGAG ATATGGAAGAACATCGATGGCGAAGGCAGCCTCCTGGGTTAACACTGACGCT CATGCACGAAAGCGTGGGGAGCAAACAGGATTAGATACCCTGGTAGTCCACG CCCTAAACGATGTCAACTAGTTGTTGGGCCTTATTAGGCTTGGTAACGAAGC TAACGCGTGAAGTTGACCGCCTGGGGAGTACGGTCGCAAGATTAAAACTCAA AGGAATTGACGGGGACCCGCACAAGCGGTGGATTATGTGGATTAATTCGATG CAACGCGAAAAACCTTACCTACCCTTGACATGTAGCGAATTTTCTAGAGAT AGATTAGTGCTTCGGGAACGCTAACACAGGTGCTGCATGGCTGTCGTCAGCT CGTGTCGTGAGATGTTGGGTTAAGTCCCGCAACGAGCGCAACCCTTGTCATT AATTGCCATCATTTGGTTGGGCACTTTAATGAGACTGCCGGTGACAAACCGG AGGAAGGTGGGGATGACGTCAAGTCCTCATGGCCCTTATGGGTAGGGCTTCA CACGTAATACAATGGCGCGTACAGAGGGTTGCCAACCCGCGAGGGGGAGCTA ATCTCAGAAAGCGCGTCGTAGTCCGGATCGGAGTCTGCAACTCGACTCCGTG AAGTCGGAATCGCTAGTAATCGCGGATCAGCATGTCGCGGTGAATACGTTCC CGGGTCTTGTACACACCGCCCGTCACACCATGGGAGTGGGTTTCACCAGAAG CAGGTAGTCTAACCGTAAGGAGGGCGCTTGCCACGGTGAGATTCATGACTGG GGTG |

**Table 2. The sequence for the AOB Type A1 ammonia-oxidizing bacterium. Represented by R7clone187. SEQ ID NO:2.**

| |
|---|
| ATTGAACGCTGGCGGCATGCTTTACACATGCAAGTCGAACGGCAGCACGGAT GCTTGCATCTGGTGGCGAGTGGCGGACGGGTGAGTAATGCATCGGAACGTAT CCAGAAGAGGGGGGTAACGCATCGAAAGATGTGCTAATACCGCATATACTC TAAGGAGGAAAGCAGGGGATCGAAAGACCTTGCGCTTTGGAGCGGCCGATG TCTGATTAGCTAGTTGGTGGGGTAAAGGCCTACCAAGGCGACGATCAGTAGT TGGTCTGAGAGGACGACCAGCCACACTGGGACTGAGACACGGCCCAGACTCC TACGGGAGGCAGCAGTGGGGAATTTTGGACAATGGGCGCAAGCCTGATCCAG CAATGCCGCGTGAGTGAAGAAGGCCTTCGGGTTGTAAAGCTCTTTCAGTCGA GAAGAAAAGGTTACGGTAAATAATCGTGACCCATGACGGTATCGACAGAAG AAGCACCGGCTAACTACGTGCCAGCAGCCGCGGTAATACGTAGGGTGCAAGC GTTAATCGGAATTACTGGGCGTAAAGGGTGCGCAGGCGGCCTTGTAAGTCAG ATGTGAAATCCCCGGGCTTAACCTGGGAATTGCGTTTGAAACTACAAGCTA GAGTGTGGCAGAGGGAGGTGGAATTCCATGTGTAGCAGTGAAATGCGTAGAG ATATGGAAGAACATCGATGGCGAAGGCAGCCTCCTGGGTTAACACTGACGCT CATGCACGAAAGCGTGGGGAGCAAACAGGATTAGATACCCTGGTAGTCCACG CCCTAAACGATGTCAACTAGTTGTTGGGCCTTATTAGGCTTGGTAACGAAGC TAACGCGTGAAGTTGACCGCCTGGGGAGTACGGTCGCAAGATTAAAACTCAA AGGAATTGACGGGGACCCGCACAAGCGGTGGATTATGTGGATTAATTCGATG CAACGCGAAAAACCTTACCTACCCTTGACATGTAGCGAATTTTCTAGAGAT AGATTAGTGCTTCGGGAACGCTAACACAGGTGCTGCATGGCTGTCGTCAGCT CGTGTCGTGAGATGTTGGGTTAAGTCCCGCAACGAGCGCAACCCTTGTCATT AATTGCCATCATTTGGTTGGGCACTTTAATGAGACTGCCGGTGACAAACCGG AGGAAGGTGGGGATGACGTCAAGTCCTCATGGCCCTTATGGGTAGGGCTTCA CACGTAATACAATGGCGCGTACAGAGGGTTGCCAACCCGCGAGGGGGAGCTA ATCTCAGAAAGCGCGTCGTAGTCCGGATCGGAGTCTGCAACTCGACTCCGTG AAGTCGGAATCGCTAGTAATCGCGGATCAGCATGTCGCGGTGAATACGTTCC CGGGTCTTGTACACACCGCCCGTCACACCATGGGAGTGGGTTTCACCAGAAG CAGGTAGTCTAACCGTAAGGAGGGCGCTTGCCACGGTGAGATTCATGACTGG GGTG |

**Table 3. The sequence for the AOB Type B ammonia-oxidizing bacterium. Represented by R3clone5. SEQ ID NO:3.**

| |
|---|
| ATTGAACGCTGGCGGCATGCTTTACACATGCAAGTCGAACGGCAGCACGGG GCAACCCTGGTGGCGAGTGGCGAACGGGTGAGTAATACATCGGAACGTATCT TCGAGGGGGGATAACGCACCGAAAGGTGTGCTAATACCGCATAATCTCCAC GGAGAAAAGCAGGGGATCGCAAGACCTTGCGCTCTTGGAGCGGCCGATGTCT GATTAGCTAGTTGGTGAGGTAATGGCTTACCAAGGCGACGATCAGTAGCTGG TCTGAGAGGACGACCAGCCACACTGGGACTGAGACACGGCCCAGACTCCTAC GGGAGGCAGCAGTGGGGAATTTTGGACAATGGGGGAAACCCTGATCCAGCCA TGCCGCGTGAGTGAAGAAGGCCTTCGGGTTGTAAAGCTCTTTCAGCCGGAAC GAAACGGTCACGGCTAATACCCGTGACTACTGACGGTACCGGAAGAAGAAG CACCGGCTAACTACGTGCCAGCAGCCGCGGTAATACGTAGGGTGCAAGCGTT AATCGGAATTACTGGGCGTAAAGCGTGCGCAGGCGGTTTTGTAAGTCAGATG TGAAAGCCCCGGGCTTAACCTGGGAACTGCGTTTGAAACTACAAGGCTAGAG TGTGGCAGAGGGGGGTGGAATTCCACGTGTAGCAGTGAAATGCGTAGAGATG TGGAGGAACACCGATGGCGAAGGCAGCCCCCTGGGTTAACACCGACGCTCAG GCACGAAAGCGTGGGGAGCAAACAGGATTAGATACCCTGGTAGTCCACGCCC TAAACGATGTCAACTAGTTGTCGGGTCTTAACGGACTTGGTAACGCAGCTAA CGCGTGAAGTTGGCCGCCTGGGGAGTACGGTCGCAAGATTAAAACTCAAAGG AATTGACGGGGACCCGCACAAGCGGTGGATTATGTGGATTAATTCGATGCAA CGCGAAAAACCTTACCTACCCTTGACATGTACCGAAGCCCGCCGAGAGGTGG GTGTGCCCGAAAGGGAGCGGTAACACAGGTGCTGCATGGCTGTCGTCAGCTC GTGTCGTGAGATGTTGGGTTAAGTCCCGCAACGAGCGCAACCCTTGTCATTA ATTGCCATCATTCAGTTGGGCACTTTAATGAAACTGCCGGTGACAAACCGGA GGAAGGTGGGGATGACGTCAAGTCCTCATGGCCCTTATGGGTAGGGCTTCAC ACGTAATACAATGGCGCGTACAGAGGGTTGCCAACCCGCGAGGGGGAGCTAA TCTCAGAAAGCGCGTCGTAGTCCGGATCGGAGTCTGCAACTCGACTCCGTGA AGTCGGAATCGCTAGTAATCGCGGATCAGCATGTCGCGGTGAATACGTTCCC GGGTCTTGTACACACCGCCCGTCACACCATGGGAGTGGGTTTCACCAGAAGC AGGTAGTCTAACCGCAAGGAGGGCGCTTGCCACGGTGAGATTCATGACTGGG GTG |

**Table 4. The sequence for the AOB Type C ammonia-oxidizing bacterium. Represented by R3clone47. SEQ ID NO:4.**

| |
|---|
| ATTGAACGCTGGCGGCATGCTTTACACATGCAAGTCGAACGGCAGCGGGGGC TTCGGCCTGCCGGCGAGTGGCGAACGGGTGAGTAATACATCGGAACGTGTCC TTAAGTGGGGAATAACGCATCGAAAGATGTGCTAATACCGCATATCTCTGA GGAGAAAAGCAGGGGATCGCAAGACCTTGCGCTAAAGGAGCGGCCGATGTCT GATTAGCTAGTTGGTGGGGTAAAGGCTTACCAAGGCAACGATCAGTAGTTGG TCTGAGAGGACGACCAACCACACTGGGACTGAGACACGGCCCAGACTCCTAC GGGAGGCAGCAGTGGGGAATTTTGGACAATGGGCGAAAGCCTGATCCAGCCA TGCCGCGTGAGTGAAGAAGGCCTTCGGGTTGTAGAGCTCTTTTAGTCAGAAA GAAAGAATCATGATGAATAATTATGATTTATGACGGTACTGACAGAAAAAG CACCGGCTAACTACGTGCCAGCAGCCGCGGTAATACGTAGGGTGCGAGCGTT AATCGGAATTACTGGGCGTAAAGGGTGCGCAGGCGGTTTTGTAAGTCAGATG TGAAAGCCCCGGGCTTAACCTGGGAATTGCGTTTGAAACTACAAGGCTAGAG TGCAGCAGAGGGGAGTGGAATTCCATGTGTAGCAGTGAAATGCGTAGAGATG TGGAAGAACACCGATGGCGAAGGCAGCTCCCTGGGTTGACACTGACGCTCAT GCACGAAAGCGTGGGGAGCAAACAGGATTAGATACCCTGGTAGTCCACGCCC TAAACGATGTCAACTGGTTGTCGGATCTAATTAAGGATTTGGTAACGTAGCT AACGCGTGAAGTTGACCGCCTGGGGAGTACGGTCGCAAGATTAAAACTCAA AGGAATTGACGGGGACCCGCACAAGCGGTGGATTATGTGGATTAATTCGATG CAACGCGAAAAACCTTACCTACCCTTGACATGCTTGGAATCTAGTGGAGAC ATAAGAGTGCCCGAAAGGGAGCCAAGACACAGGTGCTGCATGGCTGTCGTCA GCTCGTGTCGTGAGATGTTGGGTTAAGTCCCGCAACGAGCGCAACCCTTGTC ACTAATTGCTATCATTCTAAATGAGCACTTTAGTGAGACTGCCGGTGACAA ACCGGAGGAAGGTGGGGATGACGTCAAGTCCTCATGGCCCTTATGGGTAGGG CTTCACACGTAATACAATGGCGTGTACAGAGGGTTGCCAACCCGCGAGGGGG AGCCAATCTCAGAAAGCACGTCGTAGTCCGGATCGGAGTCTGCAACTCGACT CCGTGAAGTCGGAATCGCTAGTAATCGCGGATCAGCATGCCGCGGTGAATAC GTTCCCGGGTCTTGTACACACCGCCCGTCACACCATGGGAGTGGTTTCACC AGAAGCAGGTAGTTTAACCGTAAGGAGGACGCTTGCCACGGTGGGGGTCATG ACTGGGGTG |

For the purposes of the present invention, an isolated bacterial strain is one that has undergone some degree of purification from its natural environment. A culture of a bacterium is considered to be biologically pure if at least 20% of the bacteria are from one bacterial strain. However, it is preferable of the culture is at least 33% pure, more preferable if the culture is at least 45% pure and most preferable at least 90% pure.

The bacterial strain of the present invention may also be combined with each other, other species of bacteria, nutrients, and/or other components to provide a composition for maintaining or purifying water-containing media. It may be desirable, for example, to combine the bacteria of the present invention with bacteria capable of removing other pollutants or undesirable compounds from water-containing media. Examples of such bacteria include nitrite-oxidizing bacteria (chemolithoautotrophic bacteria which oxidize nitrite to nitrate), heterotrophic bacteria (which mineralize organic material into ammonia and other substances), and other bacteria which will be known to those of skill in the art. Nitrite-oxidizing bacteria are known from the *Nitrospira* group of bacteria, the alpha, gamma and delta subdivisions of the *Proteobacteria.* Examples include species of the genera *Nitrospira, Nitrospina,* and *Nitrobacter.* Nitrate-reducing bacteria are known from the genera *Azoarcus, Pseudomonas* and *Alcaligenes.* Heterotrophic bacteria are known from the genera *Bacillus, Pseudomonas,* and *Alcaligenes.* Such are available from known sources (e. g., American Type Culture Collection, 10801 University Blvd., Manassas VA 20100, USA) or could be isolated directly from aquaria biofilters.

For example, the bacterial strain of the present invention could be combined with nitrite-oxidizing bacteria such that ammonia present in the water system would be oxidized to nitrite and the nitrite oxidized to nitrate. Another example would be to combine the bacterial strain of the present invention with aerobic or anaerobic denitrifying bacteria. In this case, the nitrate which is produced by the interaction of the bacterial strains of the present invention with nitrite-oxidizing bacteria would be reduced to dinitrogen or other nitrogen based products. A third example would be to combine the bacterial strain of the present invention with heterotrophic bacteria which mineralize organic matter into simpler inorganic substances which, subsequently, can be utilized as substrates by the bacterial strains of the present invention.

The isolated bacterial strains disclosed here comprise bacteria which are similar to ammonia-oxidizing bacteria (AOB) of the beta subdivision of the *Proteobacteria.* However, the bacterial strains of SEQ ID NO: 1 and SEQ ID NO:2 can be detected by fluorescent *in situ* hybridization (FISH) to the exclusion of SEQ ID NO:3, SEQ ID NO:4 and other AOB beta subdivision *Proteobacteria* with an oligonucleotide probe having the sequence:
5'-CCC CCC TCT TCT GGA TAC 3'(SEQ ID NO : 5).

The bacterial strains of SEQ ID NO: 1 and SEQ ID NO:2 can be detected via the polymerase chain reaction (PCR) to the exclusion of SEQ ID NO:3, SEQ ID NO:4 and other AOB beta subdivision Proteobacteria with a primer set of the sequences:
forward primer 5' -CGG AAC GTA TCC AGA AGA 3 ' (SEQ ID NO : 6 ),
reverse primer 5' -ATC TCT AGA AAA TTC GCT 3' (SEQ ID NO : 7 ).

The bacterial strain of SEQ ID NO: 3, which is the subject of this invention, can be detected by fluorescent in situ hybridization (FISH) to the exclusion of SEQ ID NO:1, SEQ ID NO:2, SEQ ID NO:4 and other AOB beta subdivision *Proteobacteria* with an oligonucleotide probe having the sequence:

### 5'-TCC CCC ACT CGA AGA TAC G 3' ( SEQ ID NO:8).

The oligonucleotide probe of SEQ ID NO: 8 has one mismatch in the middle of the sequence with *Nitrosospira* AOB clade members *Nitrosospira briensis* (135505), *Nitrosospira tenuis* (m96405), and *Nitrosospira tenuis* (m96404). The stringency of the probe can be adjusted by varying the formamide concentration in the hybridization process and thus one skilled in the art should be able to distinguish between these clade members and the bacterial strain represented by SEQ ID NO: 3.

The bacterial strain of SEQ ID NO:3 can be detected via PCR to the exclusion SEQ ID NO:1, SEQ ID NO:2, SEQ ID NO: 4 and other AOB beta subdivision Proteobacteria with a set of primers with the sequences:
Forward primer 5'-ATC GGA ACG TAT CTT CG 3' (SEQ ID NO:9) and
Reverse primer 5'-CCA CCT CTC RGC GGG C 3' (SEQ ID NO:10).

The bacterial strain of SEQ ID NO:4 can be detected via PCR to the exclusion SEQ NO:1, SEQ ID NO:2, SEQ ID NO: 3 and other AOB beta subdivision Proteobacteria with a set of primers with the sequences:
Forward primer 5'-TCA GAA AGA AAG AAT CAT G 3' (SEQ ID NO:11) and
Reverse primer 5' -GTC TCC AYT AGA TTC CAA G 3 ' (SEQ ID NO:12).

The third aspect of the invention also provides a composition comprising a concentrated bacterial strain capable of oxidizing ammonia to nitrite, wherein the 16S rDNA of the bacteria has a nucleotide sequence of SEQ ID NO:3. According to the invention, the bacterial strain is considered to be concentrated if the bacterial strain occurs in a concentration which is higher than its concentration occurred in nature. In general, the concentration of the bacterial strain will be at least 20% of the total cells in the sample as determined by standard techniques such as molecular probing using fluorescent in situ hybridization techniques, which will be known to those skilled in the art, using appropriate controls and enumeration methods. More preferably, the concentration of the bacterial strain would be 33% or greater of the total cells, even more preferably 45%, and most preferably 90% or greater of the total cells. We also provide a composition where, additionally, more than one of the bacteria which have a nucleotide sequence of SEQ ID NO:1, SEQ ID NO:2, or SEQ ID NO:4 is present in the mixture. In this case, the percentage stated above relate to percentage of total AOBs in the mixture with the understanding that the balance of cell population might be comprised of nitrite-oxidizing bacteria or other types of bacteria.

It is understood that the bacterial strains, and the composition of the present invention can be in a form of powder, liquid, a frozen form, and a freeze-dried form. These are commonly referred to as "commercial additives". Such forms include, but are not limited to:
(1) a liquid form, wherein one or more strains are in a liquid solution containing inorganic salts or organic compounds such that the viability of the cells is not destroyed during the course of storage;
(2) a frozen form, wherein one or more of the strains are in a liquid mixture as above, optionally including cryoprotectant compounds to prevent cell lysis, is frozen and stored at a temperature at or below 32°F;
(3) a powder form, which has been produced by freeze-drying or other means, wherein the dehydrated form of one or more of the strains or compositions can be stored at normal room temperature without loss of viability.

Obtaining a proper form of the bacterial strain and the composition of the present invention is well within the skill in the art in view of the instant disclosure. It is also understood that the bacterial strains and the composition of the present invention can be used alone, or in combination with other components. Examples of such components include, but are not limited to, nitrite-oxidizing bacteria, heterotrophic nitrite oxidizing bacteria, heterotrophic ammonia oxidizing bacteria, and the like. All of the forms of the biologically pure bacterial strain may also contain nutrients, amino acids, vitamins and other compounds which serve to preserve and promote the growth of the bacterial strain. The bacterial strains and the compositions of the present invention can be used in freshwater aquaria, seawater aquaria, and wastewater to alleviate the accumulation of ammonia. They can also be used in a bioremediation process to reduce the level of pollution caused by the ammonia. A biomediation process, also called bioaugmentation, includes, but is not limited to, the supplemental addition of microorganisms to a system (e.g. a site where biological or chemical contamination has occurred) for the purposes of promoting or establishing biological and/or chemical processes which results in the change of one or more forms of chemical compounds present in the original system.

Accordingly, a fifth aspect of the present invention provides a method of alleviating the accumulation of ammonia in a medium. The method includes a step of placing into the medium a sufficient amount of a bacterial strain capable of oxidizing ammonia to nitrite to alleviate the accumulation of ammonia in the medium, wherein the 16S rDNA of the bacteria strains has a nucleotide sequence of 99% or greater similarity to SEQ ID NO:3. The amount of the bacterial strain is sufficient if the added bacteria can alleviate or prevent the accumulation of ammonia in the medium. In general, the addition of the bacterial strain of the invention to a freshwater or saltwater aquarium is expected to reduce the maximum ammonia concentration by at least 50% over the level which would be attained in the absence of the bacterial strain.

It will be appreciated that the actual levels achieved in a given setting will be a function of the size and contents of the systems, i.e. the number of fish, plants, etc. In a newly set-up 37 liter aquarium with 10 fish, the ammonia concentration may reach 7 mg/L or higher without addition of the bacterial strain, whereas the maximum level can be reduced to about 2 mg/L by addition of the bacterial strain. In general, the maximum ammonia concentration would not be expected to exceed 3 mg/L if the bacterial strain of the invention is added to such a system. When the system reaches a steady state, the ammonia levels drop back to below 0.5 mg/L, a process which occurs more rapidly when the bacterial strain of the invention is present.

In one embodiment of the present invention, the bacterial strain of the present invention are placed directly into a medium such as, but not limited to, freshwater aquaria, seawater aquaria, and wastewater. Preferably, the bacteria can be first grown on a rotating biological contactor and then placed in the medium. In a different embodiment, the bacteria of the present invention can be placed on a biofilter unit contained in the medium. In another embodiment the bacteria of the present invention could be immobilized in an immobilizing polymer, such as, but not limited too, acrylamide or ones constructed with alginate or carrageenan, then this bacterial-laced polymer material placed in a filter or as itself in the filter stream of the facility.

As used herein, the term "aquarium" is intended to mean a container which may be made of, in combination or in its entirety, but not limited to, glass, plastic, or wood that holds water and in which living aquatic organisms (such as fish, plants, bacteria and invertebrates) are placed, and the contents thereof. An aquarium may be for the purposes of displaying aquatic organisms, for their short or long-term holding, for scientific study, for transportation and other purposes. A freshwater aquarium is generally an aquarium in which the liquid medium has a salinity of less than 15 parts per thousand. A saltwater aquarium is generally an aquarium in which the liquid medium has a salinity of more than 15 parts per thousand. The term "aquarium water" is used to refer to the medium which is contained within the aquarium, and its associated filter systems, in which the aquatic organisms reside. Aquarium water may contain a wide range of inorganic or organic chemical substances and, therefore, may have a wide range of concentration of such parameters as in salts, pH, total dissolved solids, and temperature to name a few.

As used herein, "wastewater" generally refers to a liquid medium which is the product of an industrial or human process. It may need to be treated by one or more filtration methods to render it less harmful to the environment such that it conforms to discharge standards as determined by a governmental agency. A wastewater may also be recycled such that it is not discharged to the environment.

As used herein, a "biological filter", also called a "biofilter", generally refers to a filter type whose purpose is to promote the growth of microorganisms, or provide a substrate for the attachment and growth of microorganisms. A biofilter may be part of an aquarium filtration system or a wastewater filtration system. As used herein, the term "rotating biological contactor" generally refers to a type of biofilter which rotates in the water or medium. It may be completely or partially submerged in the water or medium. Persons skilled in the art will recognize rotating biological contactors as embodied in United States Patents Nos. 2,085,217; 2,172,067; 5,423,978; 5,419,831; 5,679,253; 5,779,885.

As used herein, "filter floss" refers to irregularly shaped natural or synthetic multi-stranded material which may serve as a biofilter, a mechanical filter or a combination of these.

As used herein, "aquarium gravel" refers to a substrate commonly placed inside, on the bottom, of an aquarium. It may be composed of irregular or regular shaped pieces of rock, coral, plastic or other material. It may serve as a biofilter, a mechanical filter, for decorative purposes or a combination of these.

As used herein, the term "filter sponge" refers to a natural or synthetic material which when used in an aquarium or as part of an aquarium filtration system may serve as a mechanical filter or a biofilter or both.

As used herein, "plastic filter media" refers to a man-made material which serves as a biofilter or mechanical filter or both. It may be plastic molded or injected molded.

Further disclosed are nucleotide probes for detecting and measuring the amount of bacteria of the present invention which are present in a medium. One probe has a nucleotide sequence of 5'-CCC CCC TCT TCT GGA TAC 3' ( SEQ ID NO:5) to detect Type A AOB with the 16S rDNA nucleotide sequence of SEQ ID NO: 1 or SEQ ID NO:2. Another probe has the sequence 5'-TCC CCC ACT CGA AGA TAC G 3' (SEQ ID NO:8) and can detect Type B AOB with the 16S rDNA nucleotide sequence of SEQ ID NO: 3. The nucleotide probes can be synthesized by methods which are known in the art.

The nucleotide probes can be labeled by any labels that are detectable. Examples of suitable labels include, but are not limited to, radioactive labels, fluorescent labels and the like. Suitable labeling materials are commercially available and would be known to those of ordinary skill in the art. The methods of labeling an oligonucleotide or a polynucleotide are also known to those of ordinary skill in the art. (See, for example, Sambrook, J., E. F. Fritsch, and T. Maniatis, Molecular Cloning - A Laboratory Manual, 2nd edition, 1989, Cold Spring Harbor Press.) The detection process can also vary and is not limited to the methods described herein. Other devices, such as DNA chips or Quantitative PCR machines, could utilize the probes described herein, therefore, these and other detection methods and devices are incorporated herein.

The nucleotide probes can hybridize with 16S rDNA of the bacterial strain of the present invention. Accordingly, the nucleotide probes are well suited for use in a method for detecting and determining the quantity of bacteria of the present invention.

We also disclosed a method for detecting and determining the quantity of bacteria capable of oxidizing ammonia to nitrite in a medium, wherein the 16S rDNA of the bacteria has a nucleotide sequence of SEQ ID NO: I, SEQ ID NO:2, or SEQ ID NO:3. The method includes the steps of:
(a) Providing a detectably labeled probe;
(b) isolating total DNA from a medium;
(c) exposing the isolated total DNA to the detectably labeled probe under conditions under which the probe hybridizes to only the nucleic acid of the bacteria, wherein the 16S rDNA of the bacteria has a nucleotide sequence of SEQ ID NO:1, SEQ ID NO:2, or SEQ ID NO:3;
(d) detecting and measuring the hybridized probe for detecting and measuring the quantity of the bacteria.

The medium can be aquarium water, wherein the DNA is isolated therefrom. The medium can also contain a material selected from the group consisting of aquarium gravel, sponge filter material, filter floss, and plastic filter media, but is not considered to be limited to these. Accordingly, the DNA can be isolated from the above and other sources where such bacteria may be expected to be found.

The detection method provides an effective tool for one to monitor and detect the occurrence of bacteria capable of oxidizing ammonia to nitrite in a medium.

The method also provides a tool for one to check the commercial additives to determine the effectiveness of the additives, particularly in freshwater aquaria, by measuring the occurrence and activity of the bacteria of the present invention.

We also disclose PCR primer sets for the detection and quantification of the bacteria of the present invention which are present in a medium. The primers sets are composed of the sequences; set 1) forward primer 5' -CGG AAC GTA TCC AGA AGA 3' (SEQ ID NO:6) and reverse primer 5' -ATC TCT AGA AAA TTC GCT 3' (SEQ ID NO:7); set 2) forward primer 5'-ATC GGA ACG TAT CTT CG 3' (SEQ ID NO:9) and reverse primer 5'-CCA CCT CTC RGC GGG C 3' (SEQ ID NO:10); set 3) forward primer 5' -TCA GAA AGA AAG AAT CAT G 3' (SEQ ID NO:11) and reverse primer 5' -GTC TCC AYT AGA TTC CAA G 3' (SEQ ID NO:12). The PCR primers can be synthesized by methods which are known in the art.

The PCR primers are well suited for use in a method for detecting and determining the quantity of bacteria of the present invention.

We also disclose a method for detecting and determining the quantity of bacteria capable of oxidizing ammonia to nitrite in a medium, wherein the 16S rDNA of the bacteria has a nucleotide sequence of SEQ ID NO: I, SEQ ID NO:2, SEQ ID NO:3 or SEQ ID NO:4. The method includes the steps of:
(a) Providing a PCR primer set;
(b) isolating total DNA from a medium;
(c) exposing the isolated total DNA to the PCR primer set under conditions under which the primers anneal specifically only to the nucleic acid of the bacteria, wherein the 16S rDNA of the bacteria has a nucleotide sequence of SEQ ID NO:1, SEQ ID NO:2, SEQ ID NO:3 or SEQ ID NO:4.

### MATERIALS AND METHODS

A series of assays and experiments were conducted to isolate, identify and show the efficacy of the bacterial strains reported herein. They involved a variety of bacterial culturing techniques, molecular biological analyses of DNA extracted from samples of the cultures, molecular biological analysis of the bacterial strains, and the application of concentrated cultures of the bacterial strains to aquaria to measure their ability to control ammonia concentrations.

**Bacteria Culturing.** A number of bacterial culturing vessels (termed reactors) were constructed and seeded with bacterial biomass gathered from operating aquaria. Each reactor received 4.95 L of a mineral salt solution (made up in distilled water) containing 50g KH₂PO₄, 50g K₂HPO₄, 18.75g MgSO₄•7H₂O, 1.25g CaCl₂•2H₂O and 1g FeSO₄•7H₂O. Air was provided such that the dissolved oxygen was equal to or greater than 7.5 mg/L, stirring was provided, and the reactors were kept in a darkened cabinet at approximately 28°C.

The ammonia and nitrite concentrations were measured daily using flow injection analysis (FIA, Tecator FIAStar 5010 system) while pH was determined with an electrode (Denver Instruments Model 225 pH/ISE meter and associated pH/ATC electrode). Nitrate and conductivity were measured periodically and the data were used to determine when water changes were required. Bacterial biomass was retained in the reactors during water changes because the biomass is very floccular in nature. Thus prior to decanting 50% of the reactor's volume through the appropriate sampling port the biomass was settled by turning off the air and stirring mechanism for 1 hour. Additionally, reactors were occasionally scrubbed to remove the biomass from the surfaces to return the biomass to suspension. Microbiological samples were taken routinely for DNA extraction (for PCR) and cell fixation (for FISH) for further analysis.

**Nucleic acid sampling and extraction.** For DNA extraction, samples of appropriate biological filtration medium were taken and resuspended in cell lysis buffer (40 mM EDTA. 50 mM Tris-HCI, pH 8.3). Samples were stored at -20°C or -74°C until extraction. For processing, lysozyme was added to the samples to a final concentration of 10 mg/ml. After incubation at 37°C for 90 minutes, 20% sodium dodecyl sulfate (SDS) was added to a final concentration of 1%. Then the samples were subjected to four freeze/thaw cycles followed by the addition of proteinase K (stock concentration, 10 mg/ml) to a final concentration of 2 mg/ml and incubated at 70°C for 35 minutes. In some cases, additional proteinase-K and SDS were added and the sample was incubated at 55°C for another 30 minutes.

After cell lysis, DNA was extracted using Easy DNA extraction kit (Qiagen Inc., Santa Clarita, CA). DNA was eluted to a 50 µl volume and quantified by Hoechst type 33258 dye binding and fluorometry (DynaQuant 200, Hoefer Pharmacia Biotech Inc., San Francisco, Calif. ).

**Clone libraries of PCR amplified rRNA genes.** Clone libraries were derived from DNA extracts from biomass samples taken from reactors and aquaria. Bacterial ribosomal RNA gene fragments were amplified with the primers S-D-Bact-0011-a-S-17 (8f; GTT TGA TCC TGG CTC AG) (SEQ ID NO:13) and 1492r (eubacterial; GGT TAC CTT GTT ACG ACT T) (SEQ ID NO:14). PCR conditions, cycle parameters, and reaction components were as previously described (DeLong, E. F. 1992. Archaea in coastal marine environments Proc. Natl. Acad. Sci. USA 89: 5685 - 5689.) PCR products were evaluated by agarose gel electrophoresis. PCR fragments were cloned with a TA Cloning kit (Invitrogen, Carlsbad, Calif.), as described in the manufacturer's directions, after rinsing with TE buffer and concentrating to 30 µl with a Centricon® concentrator (Amicon, Inc. Beverly, MA).

**Sequencing and phylogenetic analysis.** The 16S rDNA insert from each clone that comprised the clone library were screened by restriction enzyme analysis (REA) using the restriction enzyme Hae III in order to A) ensure that the 16S rDNA insert was amplifiable and B) determine whether the 16S rDNA possessed a unique REA pattern when digested with the Hae III enzyme. If a clone was amplifiable and possessed a unique REA pattern, then the clone's plasmid containing the 16S rDNA insert of interest was partially sequenced. The amplified PCR 16S rDNA template of each clone selected for sequencing was cleaned using the PCR Purification Kit Catalog No. 28142 (Qiagen, Santa Clarita, CA). Sequencing was performed using a LiCor 4000L automated DNA sequencer on template cycle-sequenced with fluorscently labelled primers and SequiTherm EXCEL^{™}II DNA Sequencing kits (Epicentre Technologies, Madison, WI). Up to two or three clones of the same REA pattern were partially sequenced to ensure that they were identical. Many clones, but especially those affiliated with the *Nitroso-* clade of the beta subdivision of the class *Proteobacteria,* were fully sequenced and phylogentically analyzed by PAUP (Phylogenetic Analysis Using Parsimony ver 4.0b2a, D.L. Swofford) (bootstrap values and distance analysis), ARB (A Software Environment for Sequence Date, W. Ludwig and O. Strunk) (phylogenetic tree) and Phylip (Phylogeny Inference Package J. Felsentein) (similarity matrix). Primers and probes for the clone of interest from the clone libraries were developed using ARB probe design and probe match programs as well as after manual alignment. Primers and probes were double checked with BLAST (Altschul, S. F., W. Gish, W. Miller, E. W. Myers, and D. J. Lipman. 1990. Basic local alignment tool. J. Mol. Biol. 215:403-410). The specificity of the primers was determined by using them on DNA extracted from clones and pure cultures of known bacteria. The specificity of the probes was tested using pure cultures of known bacteria and samples from the reactors.

**DGGE analysis and profiling.** For general eubacterial DGGE analysis, rDNA fragments were amplified using the forward 358f (eubacterial;CCT ACG GGA GGC AGC AG) (SEQ ID No:15) with a 40-bp GC-clamp on the 5' end as described by Murray et al. (Murray, A. L., J. T.Hollibaugh, and C. Orrego. 1996. Phylogenetic compositions of bacterioplankton from two California estuaries compared by denaturing gradient gel electrophoresis of 16S rDNA fragments. Appl. Environ. Microbiol. 62:2676-2680), and the reverse primer S-*-Univ-0519-a-A-18 (519r: GWA TTA CCG CGG CKG CTG) (SEQ ID NO:16). For specific AOB DGGE, the forward primer of 358f (eubacterial; CCT ACG GGA GGC AGC AG) (SEQ ID No:15) with a 40-bp GC-clamp on the 5' end was used with the reverse primer S-*-Ntros-0639-a-A-20 (Nitroso4e: CAC TCT AGC YTT GTA GTT TC) (SEQ ID NO:17). The PCR conditions were the same and were performed on a Stratagene Robocycler Gradient 96 (La Jolla, Calif.) using the Qiagen TAQ PCR core kit (Qiagen, Santa Clarita, Calif.). PCR conditions included a hot start (80°C) and a touchdown procedure. Initial denaturation at 94°C for 3 minutes was followed by a denaturation at 94°C for 1 min. a touchdown annealing from 65°C to 55°C for 1 min 29 sec (the annealing time during the touchdown increased by 1.4 sec per cycle), and primer extension at 72°C for 56 sec (the extension time was increased 1.4 seconds per cycle). The final temperature series of the above thermal cycle was repeated for 20 total cycles, followed by a final extension at 72°C for 5 min. Amplicons were examined by agarose gel electrophoresis. DGGE was performed with a Bio-Rad D-GENE System (Bio-Rad Laboratories, Hercules, Calif.). Gels were 8. 5% acrylamide/Bis using Bio-Rad reagents (D GENE Electrophoresis Reagent Kit, Bio Rad Laboratories, Hercules, Calif.). Gel gradients were poured using Bio-Rad reagents (D GENE Electrophoresis Reagent Kit, Bio Rad Laboratories. Hercules, Calif.) with a denaturing gradient of 20 to 60% (where 100% denaturant is a mixture of 40% deionized formamide and 7 M urea) and the Bio-Rad gradient delivery system (Model 475, Bio Rad Laboratories, Hercules, Calif.). All gels were run at 200 volts for 6 hours. Gels were visualized in one of two ways. For visualization and recovery of discrete DNA bands, gels were first stained for 10 minutes in 250 ml of 1X TAE buffer in which 100 µl of ethidium bromide (1mg/ml) was added, then washed for 10 minutes in 1 X TAE buffer. For documentation purposes some gels were stained in Vistra Green (diluted 1 : 10,000) (Molecular Dynamics, Sunnyvale, Calif.) for 20 minutes, followed by a 20 minute wash in 1 X TAE buffer, and then scanned using a FluorImager SI (Molecular Dynamics, Sunnyvale, Calif.).

Individual bands were excised from the DGGE gels using alcohol sterilized scalpels. Extraction of DNA from the gel followed the methods of Ferris et al. (Ferris, M. J.. G. Muyzer, and D. M. Ward. 1996. Denaturing gradient gel electrophoresis profiles of 16S rRNA-defined population inhabiting a hot spring microbial mat community. Appl.Environ. Microbiol. 62: 340-346.). The excised band was placed in a sterile 2 ml screw cap tube with 500 µl sterile deionized water. The tubes were half-filled with glass beads (cat. no.11079-101, Biospec Products Inc., Bartlesville, Okla.) and placed in a mechanical bead beater (Mini-beadbeater-8, Biospec Products Inc., Bartlesville, Okla.) for 3 minutes at the highest setting. The processed DNA remained in the tubes at 4°C overnight. After overnight storage, the tubes were centrifuged at 3,200 X g for 8 minutes at 4°C to concentrate the gel fragments. The supernatant was transferred to a clean eppendorf tube.

To check the extraction efficiency, the supernatant was reamplified with the DGGE primers and re-analyzed by DGGE. An extraction was considered acceptable if it yielded a single band in DGGE analysis which co-migrated with the original DGGE band in the mixed population sample. The nucleotide sequence of the excised band was sequenced by the previously described methods using fluorescently labelled primers.

**Oligonucleotide probe, PCR primer development, and FISH hybridization procedures.** Oligonucleotide probes were designed that specifically hybridize with the 16S rRNA gene sequence isolated from three closely related bacteria from reactors in this study. One probe (S-G-Nsspa-0149-a-A-18) (SEQ ID NO:5) targets two reactor-derived *Nitrosospira-like* bacteria, which are represented by the sequences of SEQ ID NO:1 and SEQ ID NO:2 to the exclusion of other beta subdivision Proteobacterial ammonia-oxidizers including the sequences represented by SEQ ID NO:3 and SEQ ID NO:4. A second probe (S-G-Nsspa-0149-a-A-19) (SEQ ID NO:8) targets one reactor-derived *Nitrosospira-like* bacterium, which is represented by the sequence of SEQ ID NO:3, to the exclusion of SEQ ID NO:1, SEQ ID NO:2, SEQ ID NO:4 and other beta subdivision Proteobacterial ammonia-oxidizers. Probe matches were initially screened using BLAST (Altschul, S. F., W. Gish, W. Miller, E. W. Myers, and D. J. Lipman. 1990. Basic local alignment tool. J. Mol. Biol. 215:403-410) and CHECK_PROBE (Maidak, B.L., N. Larsen, M. J. Mccaughey, R. Overbeek, G. J. Olsen, K. Fogel, J. Blandy, and C. R. Woese. 1994. The ribosomal database project. Nucleic Acids Res. 22:3485-3487.). Probes were synthesized by Operon Tech, Inc. (Alameda, Calif.). The nucleotide sequence and position of the probes are shown in Table 5.

**TABLE 5. The nucleotide sequences and positions of oligonucleotide probes and PCR primer sets for ammonia-oxidizing bacteria.**

| Probe | Position Nucleotides | Base Sequence (5' to 3') | % formamide | Annealing Temp (°C) | Target Group |
|---|---|---|---|---|---|
| S-G-Nsspa-0149-a-A-18 | | CCC CCC TCT TCT GGC TAC | 30 | -- | SEQ ID NO:1 & SEQ ID NO:2 |
| | | | | | |
| S-G-Nsspa-0149-a-A-a9 | | TCC CCC ACT CGA AGA TAC G | 20 | -- | SEQ ID NO:3 |
| | | | | | |
| Forward primer | | CGG AAC GTA TCC AGA AGA | -- | 54 | SEQ ID NO:1 & SEQ ID NO:2 |
| | | | | | |
| Reverse primer | | ATC TCT AGA AAA TTC GCT | -- | | |
| | | | | | |
| Forward primer Reverse primer | | ATC GGA ACG TAT CTT CG CCA CCT CTC RGC GGG C | -- | 56 | SEQ ID NO:3 |
| | | | | | |
| Forward primer | | TCA GAA AGA AAG AAT CAT G | -- | 56 | SEQ ID NO:4 |
| Reverse primer | | GTC TCC AYT AGA TTC CAA G | -- | | |

The stringency for the probes (SEQ ID NO:5 and SEQ ID NO:8) was determined though a series of FISH experiments at differing formamide concentrations using the reactor biomass as a positive control for the bacterial sequences herein (SEQ ID NO:1, SEQ ID NO:2 or SEQ ID NO:3). The specificity of the probes was examined by testing against negative control cells of pure cultures of other beta subdivision ammonia-oxidizing bacteria (*Nitrosomonas europaea, Nitrosospira multiformis* and *Nitrosomonas cryotolerans*). In situ hybridization of the fixed, immobilized cells was carried out in a hybridization solution consisting of 0.9 M NaCl, 20 mM Tris/HCl (pH 7.4), 0.01% sodium dodecyl sulphate (SDS), 25 ng of oligonucleotide probe, and varying amounts of formamide. Slides were incubated in an equilibrated humidity chamber at 46°C for 90 to 120 min. The hybridization solution was rinsed off with a prewarmed (48°C) wash solution. The slides were then incubated in the wash solution for 15 min at 48°C. To achieve the same stringency during the washing step, as in the hybridization step, the wash solution contained 20mM Tris/HCl (pH 7.4), 0.01% SDS, 5 mM EDTA, and NaCl. The concentration of NaCl varied according to the percent formamide used in the solution. For 20% formamide the NaCl concentration was 215 mM, for 30% it was 120 mM, and for 40% the NaCl concentration was 46 mM. Cells were detected using a Zeiss Axioskop 2 epifluorescence microscope (Carl Zeiss, Jena, Germany) fitted with filter sets for FITC/FLUO3 and HQ CY3. The optimum stringency was determined to be 30% formamide for the S-G-Nsspa-0149-a-A-18 probe. For the S-G-Nsspa-0149-a-A-19 probe the optimum stringency was determined to be 20% formamide.

PCR: Two sets of PCR primers were developed which specifically detect *Nitrosospira-*like bacteria of the sequences report here. A third set of PCR primers was developed which specifically detects *Nitrosomonas*-like bacteria of the sequences report here. One set (SEQ ID NO:6 and SEQ ID NO:7) specifically detects *Nitrosospira-*like bacteria with the sequence SEQ ID NO:1 and sequence SEQ ID NO:2 to the exclusion of other ammonia-oxidizing bacteria (Table 6). The second set (SEQ ID NO:9 and SEQ ID NO:10) specifically detects the *Nitrosospira-*like bacteria with the sequence SEQ ID NO:3 to the exclusion of other ammonia-oxidizing bacteria (Table 6). The third set (SEQ ID NO:11 and SEQ ID NO:12) specifically detects the *Nitrosomonas-*like bacteria with the sequence SEQ ID NO:4 to the exclusion of other ammonia-oxidizing bacteria (Table 6). PCR conditions were as previously described except the annealing temperature was modified.

**Table 6. Results of the PCR primer development specificity testing and annealing temperature experiments.**

| Clone Number or Bacteria Species | Type A AOB PCR SEQ ID NO 6 & 7 | | | | Type B AOB PCR SEQ ID NO 9 & 10 | | | | Type C AOB PCR SEQ ID NO 11 & 12 | | | | |
|---|---|---|---|---|---|---|---|---|---|---|---|---|---|
| Annealing Temp. (°C) | 48 | 50 | 52 | 54 | 54 | 56 | 58 | 60 | 48 | 50 | 52 | 54 | 56 |
| R7c140 (TypeA) | + | + | + | + | - | - | - | - | - | - | - | - | - |
| R7c187 (TypeA) | + | + | + | + | - | - | - | - | - | - | - | - | - |
| R3c5 (TypeB) | - | - | - | - | + | + | + | + | - | - | - | - | - |
| R5c20 (TypeB) | - | - | - | - | + | + | + | + | - | - | - | - | - |
| R3c12 (TypeC) | - | - | - | - | - | - | - | - | + | + | + | + | + |
| RSc47 (TypeC) | - | - | - | - | - | - | - | - | + | + | + | + | + |
| N. europaea | - | - | - | - | - | - | - | - | - | - | - | - | - |
| N. multiformis | - | - | - | - | - | - | - | - | +/- | +/- | +/- | - | - |
| N. cryotolerans | - | - | - | - | - | - | - | - | - | +/- | + | +/- | - |
| Negative control | - | - | - | - | - | - | - | - | - | - | - | - | - |

| | | | | | | | | | | | | | |
|---|---|---|---|---|---|---|---|---|---|---|---|---|---|
| +/- = weak + = strong - = no signal | | | | | | | | | | | | | |

The specificity of each primer set was optimized by conducting a PCR experiment with each primer set using the temperature gradient mode of the Stratagene Robocycler. In this mode one can run a single experiment of all the reactions at up to 12 different annealing temperatures. Typically, the experiments were conducted at 4 to 6 different temperatures with 2°C increasing interval. Each PCR primer set was tested against clone product with a nucleotide sequence of SEQ ID NO: I, SEQ ID NO:2, SEQ ID NO:3, or SEQ ID NO:4. rDNA extracted from pure cultures of *Nitrosomonas europaea, Nitrosolobus multiformis* and *Nitrosomonas cryotolerans* were also tested. Table 5 presents the PCR primer sets, and the optimal annealing temperature results are shown in Table 6.

### RESULTS AND DISCUSSION

Nine clone libraries were constructed from a number of freshwater nitrifying biomasses in order to determine the identity of the ammonia oxidizer(s) responsible for oxidation of ammonia to nitrite. Details about the biomasses are presented in Table 7.

**Table 7. Details regarding the reactors and aquaria from which biomass was extracted and clone libraries was constructed.**

| **Clone library** | **Details of nitrifying biomass** |
|---|---|
| Biofarm 16 | This biomass was retrieved from the sump of BF16. The biofarm was routinely dosed 300mg/L/hr of ammonia (NH₃-N) for 6 hours per day. |
| BC5 | This biomass was kept in an aquarium (seeded from a freshwater biofarm) and dosed 5mg/L or less of ammonia every two or three days. The aquarium was not aerated. |
| BC5(2) | Same as BC5 (above). |
| R3 | This was seeded from an enriched ammonia oxidizing culture (approx 1000mg/L NH₃-N) that had been stored for 11 months. Grown at 5mg/L NH₃-N and aerated. |
| R7 | This was seeded from R1 which had been seeded from BC5. Both R1 and R7 were kept below 5mg/L ammonia (NH₃-N) and aerated. |
| R7BA6 | This biomass was recovered from a Bacterial Additive test that was inoculated with R7 biomass. |
| R5 | This biomass was derived from the biofarm feed microfilter. It was exposed to extremely high concentrations of ammonia (>500mg/L NH₃-N). The reactor was operated at 30mg/L ammonia (NH₃-N) and aerated. |
| R17 | This biomass was derived from R7 but fed at 30mg/L for a period of three weeks before being returned to 5mg/L ammonia, aerated. |
| R13 | This biomass was derived from the BC5 biomass but did not appear to have any Nitroso- bacteria by using general AOB primers. It was grown at 5 mg/L (NH₃-N) and aerated. |

The clone library data show that there are three groups of ammonia oxidizing bacteria that exist in the low ammonia feed reactors (e.g., R3, R7). Not all three AOB types were found to exist in every reactor though. The three bacteria are represented by three AOB clone groups - AOB Type A (SEQ ID NO:1) (and a subtype A1 (SEQ ID NO:2)), AOB Type B (SEQ ID NO:3). A fourth clonal type was found in high ammonia feed reactors - AOB Type C (SEQ ID NO:4).

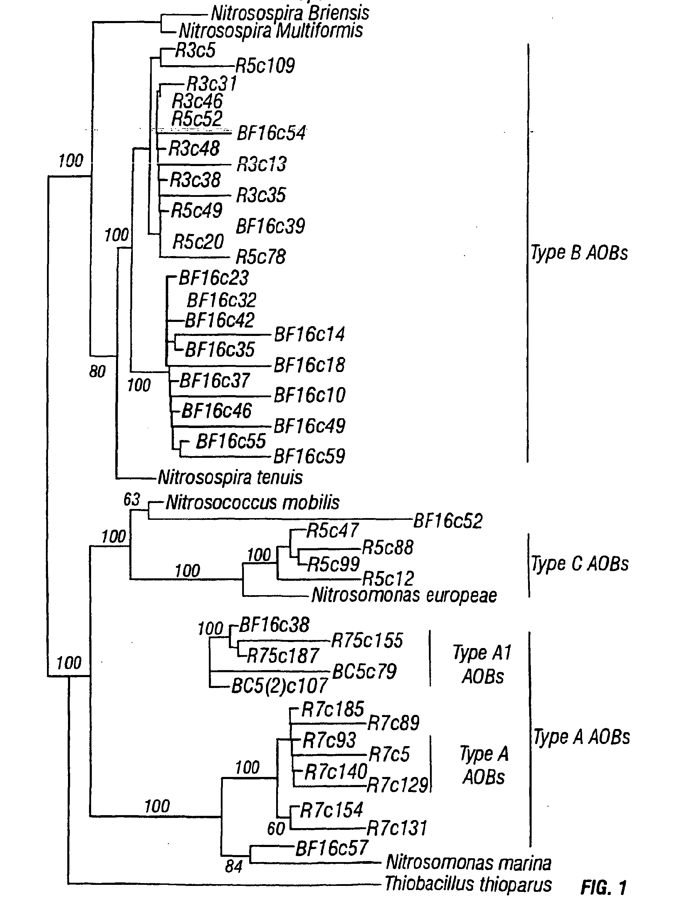

A similarity ranking was conducted for the four clonal sequences using RDP (Maidak, B.L., J. R. Cole, C. T. Parker, Jr, G. M. Garrity, N. Larsen, B. Li, T. G. Lilbum, M. J. McCaughey, G. J. Olsen, R. Overbeek, S. Pramanik, T. M. Schmidt, J. M. Tiedje and C. R. Woese. A new version of the RDP (Ribosomal Database Project). Nucleic Acids Res. 27:171-173 (1999)) (Table 8). The similarity analysis showed that AOB Type A (SEQ ID NO:1) and Type A1 (SEQ ID NO:2) are 99.6% similar. This agrees with the 16S rDNA data which showed there to be 5 mismatches in the 16Sr DNA between the type sequence for Type A (SEQ ID NO:1) and the type sequence for Type A1 (SEQ ID NO:2). The similarity analysis showed that the Type A and A1 sequences are significantly different from known AOBs of either the *Nitrosospira* or *Nitrosomonas* clades (Table 8). This results is further supported by the Bootstrap analysis which shows that the AOB Type A (SEQ ID NO:1) and Type A1 (SEQ ID NO:2) cluster together in a group that is distinct from either the *Nitrosospira* or *Nitrosomonas* clades (Fig. 1). Thus the bacteria represented by AOB Type A (SEQ ID NO:1) and Type A1 (SEQ ID NO:2) are at least new species.

**TABLE 8. Similarity ranking for the ammonia-oxidizing clones Isolated from reactors and aquaria**

| % Similarity to rDNA of: | | | | | | | | | | |
|---|---|---|---|---|---|---|---|---|---|---|
| | Type | Type | Type | Type | *Nitro* | *Nitro* | *Nitro* | *Nitro* | *Nitro* | *Nitro* |
| | A | A1 | B | C | *somo* | *sovib* | *solob* | *sospi* | *somo* | *sococ* |
| | *Nitro* | *Nitro* | *Nitro* | *Nitros* | *nas* | *rio* | *us* | *ra* | *nas* | *cus* |
| | *sospi* | *sospi* | *sospi* | *omon* | *mari* | *tenui* | *multi* | *brien* | *euro* | *mobil* |
| | *ra-* | *ra-* | *ra-* | *as-* | *na* | *s* | *formi* | *sis* | *paea* | *is* |
| rDNA source | like | like | like | like | | | s | | | |
| | | | | | | | | | | |
| Type A *Nitrosospira-* | .-- | | | | | | | | | |
| like | | | | | | | | | | |
| Type A1 | 0.99 | .-- | | | | | | | | |
| *Nitrosospira-like* | 6 | | | | | | | | | |
| Type B *Nitrosospira-* | 0.94 | 0.94 | .-- | | | | | | | |
| like | 4 | 2 | | | | | | | | |
| Type C | 0.93 | 0.93 | 0.92 | .-- - | | | | | | |
| *Nitrosomonas*-like | 4 | 2 | 5 | | | | | | | |
| *Nitrosomonas marina* | 0.95 | 0.95 | 0.92 | 0.932 | .-- | | | | | |
| | 4 | 5 | 8 | | | | | | | |
| *Nitrosovibrio tenuis* | 0.94 | 0.94 | 0.98 | 0.926 | 0.93 | .-- | | | | |
| | 8 | 6 | 8 | | 2 | | | | | |
| *Nitrosolobus* | 0.94 | 0.94 | 0.98 | 0.927 | 0.93 | 0.98 | .-- | | | |
| *multiformis* | 8 | 6 | 4 | | 7 | 9 | | | | |
| *Nitrosospira briensis* | 0.94 | 0.94 | 0.97 | 0.919 | 0.93 | 0.97 | 0.98 | .-- | | |
| | 1 | 0 | 1 | | | 9 | 0 | | | |
| *Nitrosomonas* | 0.93 | 0.93 | 0.92 | 0.984 | 0.93 | 0.93 | 0.93 | 0.92 | .-- | |
| *europaea* | 6 | 5 | 5 | | 2 | 1 | 3 | 5 | | |
| *Nitrosococcus mobilis* | 0.94 | 0.93 | 0.92 | 0.962 | 0.93 | 0.92 | 0.93 | 0.93 | 0.96 | .-- |
| | 2 | 9 | 1 | | 0 | 8 | 1 | 0 | 2 | |

The similarity analysis for the AOB Type B (SEQ ID NO:3), which is the subject of this invention, shows that this bacterium falls into *Nitrosospira* clade of AOB (Table 8). Bootstrap analysis confirms this results (Fig. 1). However, the organism is distinct enough from the closest *Nitrosospira* AOB (*Nitrosovibrio tenuis*) that it may be considered as a new species.

The similarity analysis for the AOB Type C (SEQ ID NO:4) shows that this bacterium falls into *Nitrosomonas* clade of AOB (Table 8). Bootstrap analysis confirms this results (Fig. 1). However, the organism is distinct enough from the closest *Nitrosomonas* AOB (*Nitrosomonas europaea*) that it may be considered as a new species.

The similarity rankings given in Table 8 are a guide to determining the uniqueness of one bacterial strain to another. There are no hard and fast rules regarding what percentage constitutes a new species. However, *Nitrosolobus multiformis* and *Nitrosovibrio tenuis* which have a similarity ranking of 0.989 are recognized by all microbiological authorities as distinct species, as are *Nitrosolobus multiformis* and *Nitrosospira briensis* (similarity ranking of 0.980). Since the similarity values of the bacterial strains reported herein are not higher than those for the above mentioned species pairs this is further evidence that the strains herein are novel and unique.

Therefore, the totality of the clone data, the PCR results, the phylogenetic analysis, the DGGE data and similarity ranking demonstrate that the bacterial strains reported herein are unique and distinct from known ammonia-oxidizing bacteria. Further, we expect that additional work in micro (or specialized) environments such as presented herein will result in the discovery of additional AOB related to the strains reported herein.

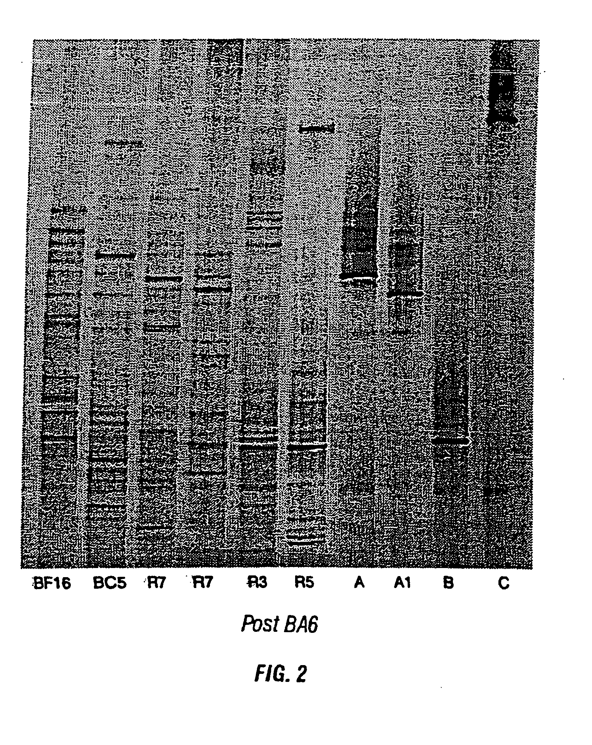

Clonal members of Type A AOB (SEQ ID NO:1 and SEQ ID NO:2) were found in both the BF16 biomass (9% of clone library) and the BC5 biomass (1-2% of clone library) (Fig. 2). The BC5 biomass was used to seed the low concentration ammonia reactor (R1), which was used to seed R7. The R7 clone library generated from the R7 biomass containing only Type A AOB clones (SEQ ID NO:1 and SEQ ID NO:2) (7% of the clone library) (Fig. 2). Hence Type A AOB bacteria have been successfully subcultured from the freshwater Biofarm to the BC5 tank and then in the R7 reactor via the R1 reactor. This demonstrates the ability to successively culture the bacteria and to maintain a viable culture of AOB with the sequences herein. Further, it demonstrates the ability to selectively enrich for the Type A AOB as the percent of this bacterium increased from 1-2% in the BC5 clone library to 7% in the R7 library.

Outwardly, the operation of the three systems (BioFarm 16, BC5 tank, and R7 reactor) would appear to be quite different (see Table 7). However, there is a common set of physicochemical conditions that may explain the presence of Type A AOBs in these systems. Although the Biofarm receives high concentrations of ammonia initially, it is allowed a period of time for the ammonia concentrations to fall to low levels (below 5mg/L NH₃-N), thus allowing the Type A bacteria to be retained in the system, exploiting a particular physiological niche of being able to grow at very low ammonia concentrations (<5mg/L NH₃-N). Similarly, the BC5 tank and the R7 reactor were both fed and maintained at ammonia levels at or below 5 mg/L NH₃-N. The Type A AOB bacteria may be able to exist at ammonia concentrations above 5mg/l NH₃-N but it is apparent that at higher concentrations of ammonia they are outcompeted by other types of AOBs (i.e., Type B (SEQ ID NO:3) and/or Type C (SEQ ID NO:4)) as evidenced by these types of AOB being present, and Type A AOB being absent, in the reactors maintained at high ammonia concentrations (Table 9) (Fig. 2).

The R7 biomass did particularly well in the bacterial additives test VI (BA6) and VII (BA7) (discussed below) as did a biomass grown in the same fashion (R19) and with the same seed (R1) in bacterial additives test VIII (BA8) (R19). Type A AOBs have been found in a number of reactors and a number of Post BA test biomasses both by specific Type A AOB PCR and FISH tests (Table 9) (Fig. 2).

Therefore, these two newly discovered bacteria Type A AOB (SEQ ID NO:1) and Type A1 (SEQ ID NO:2) predominate in low ammonia concentration environments, such as an aquarium; and, when added to such an environment in a more purified state than they naturally occur, can accelerate the establishment of ammonia oxidation in such an environment (discussed below).

Clonal members of Type B were found in the freshwater BioFarm biomasses (e.g., BF 16 - 34% of clone library) used to seed the BC tanks (BC5). Type B AOB bacteria were absent in the BC5 and R7 clone libraries, indicating that these AOBs may be more suited to the high ammonia conditions and feeding regime of a Biofarm (Fig. 2). Type B AOBs were also found in the R3 clone library (19% of clone library) (Fig. 2). The history of the R3 reactor is that it's biomass was initially enriched at high ammonia concentrations (3000mg/L NH₃-N), stored for 11 months and then matured in the reactor at low ammonia concentrations (5mg/L NH₃-N) for an extended period of time. During the initial culturing period it was likely that the ammonia concentrations would decrease over time - thus encouraging the growth of Type C and/or Type B AOBs over Type A AOBs. During the eleven months of storage the ammonia would be likely to be exhausted possibly encouraging the maintenance of Type B AOB bacteria in the system and the survival of residual Type A AOBs that had survived during the culturing phase. Finally during the maturation period in the reactor, the Type B AOB bacteria would be able to be maintained, Type A AOBs would be enriched and any residual Type C that had been originally selected for in the original culturing phase would be outcompeted and disappear.

In comparison, the Biofarm's biomass receives a relatively high concentration of ammonia for a set period of time and then allowed to gradually deplete this over time, creating both a gradient of high to low ammonia concentrations (encouraging the growth of Type B AOBs), often reaching zero thus allowing a window for the growth of Type A AOBs - low ammonia concentrations. This is a more rapid cycle (daily) than the culturing phase of the R3 biomass, but none the less consistent with a change of conditions from high to low ammonia concentrations within the biomass. Thus the gradient of ammonia concentrations in the Biofarm's biomass encourages the enrichment of a range of AOB types as confirmed by the clone library data and the results of the DGGE tests.

Type B AOBs have been found in a number of reactors and a number of Post BA test biomasses both by specific Type B AOB PCR, DGGE and FISH. However, it has not been found in as many post bacterial additive tests or clone libraries as Type A AOB (Table 9). It seems to be that if Type A AOB was inoculated into a test it was often recovered whereas Type B AOBs were only recovered in systems where Type A AOBs were not originally in the innoculum. Therefore, Type A AOBs are preferentially grown in the systems when they are present but Type B AOBs will suffice when Type A AOBs are absent.

While Type A AOBs are the most important member of a successful AOB nitrifying community for low ammonia environments such as aquarium, they are not the only AOB present. Other AOB, such as Type B (SEQ ID:3), may be necessary for the system to efficiently cope with fluctuating concentrations of ammonia even over short (days) periods of time.

Type C AOBs are not desirable as an AOB in a bacterial additive for the low ammonia concentrations typically found in an aquarium. Type C AOB bacteria were not found in the BF16, BC5 or R7 clone libraries which are low ammonia concentration environments, indicating that they were likely grown under conditions other than that found in these three environments (Fig. 2). Type C bacteria were found in the R5, R3 and R17 clone libraries (Fig. 2). The R5 biomass was grown consistently at high concentrations (30mg/L NH₃-N) and its seed was from a very high ammonia concentration (>500mg/L NH₃-N), R3's biomass had been originally grown at a high ammonia concentration before being moved to a lower ammonia concentration (5mg/L NH₃-N) and the R17 biomass was moved from a low (5mg/L NH₃-N) to a high ammonia concentration (30mg/L NH₃-N) and then back again.

The R5 biomass had been enriched at high ammonia concentrations for a long period of time even before being transferred to the R5 reactor, in effect excluding the growth of any Type A AOB bacteria as the concentration of ammonia never dropped to low levels in the feed microfilter. When the biomass was transferred to R5, and the concentration of ammonia was allowed to be reduced to lower ammonia levels, it allowed for Type B bacteria to be enriched for. The Type C bacteria would represent the bacteria enriched for initially in the microfilter and then remained in the R5 biomass when the feed was kept at relatively high ammonia concentrations (30mg/L NH₃-N).

The R3 biomass had been initially allowed to grow at high ammonia concentrations but over time the ammonia would become exhausted. This regime initially encourages the growth of Type C AOBs (at higher ammonia concentrations) and Type B AOBs (as the ammonia was utilized). Further, these pressures would not allow for the enrichment of Type A AOB which are dependent on consistently low levels of ammonia. During the operation of the R3 reactor at lower ammonia concentrations, Type C AOB bacteria would be enriched against and Type B would still survive but since Type A AOB bacteria were originally minimized in the initial enrichment there would be very few left to take advantage of the new conditions within the reactor. Therefore, Type B AOB would be expected to be the dominant AOB in this environment.

The R17 biomass shows typically what not to do for culturing Type A and/or B AOBs. The R17 biomass was derived from the R7 biomass but cultured for 3 weeks at elevated ammonia (30mg/L NH₃-N) concentrations to see if a shift in the microbial community would occur. A shift did occur and Type C AOBs became dominant, as demonstrated by the results of FISH, PCR and DGGE experiments. Furthermore, the shift was irreversible. After moving the biomass back to a low ammonia concentration (5mg/L NH₃-N) environment, the Type C AOB still remained the dominant AOB while Type A and Type B AOBs could not be detected by either FISH or DGGE. This suggests that during the three week period Type A and B AOBs were excluded from the R17 biomass. The R17 biomass did poorly in the subsequent BA VIII test suggesting that Type C AOBs are not the correct type of AOB required for an effective bacterial additive to be used in the relatively low ammonia environment of an aquarium. This conclusion is further supported by the results of the bacterial additive tests which showed that existing commercial bacterial mixtures which contain *Nitrosomonas* clade AOBs are not effective for accelerating the establishment of nitrification in aquaria (discussed below).

The Type C bacteria are very closely related phylogenetically to those bacteria that have been found in wastewater treatment plants which also receive ammonia concentrations of around 30mg/L NH₃-N (similar to R5).

The PCR primer sets described herein were used to detect the presence or absence of the AOB strains reported here in a variety of environments. The environments include pre bacterial additive test mixtures, post bacterial additive test aquaria filters, and commercial mixtures of nitrifying bacteria manufactured and sold by other companies. In addition, DNA extracted from the pure culture of other AOB was tested.

The results of these experiments are summarized in Table 9. The data show that the PCR primer groups are specific for the bacterial strain reported herein and allow one to detect each strain exclusive of the other strains. Further, pure cultures of known AOB are not amplified with any of the PCR primer sets reported herein. This demonstrates that the bacteria reported herein can be distinguished from known AOB.

The data also show that one would expect the commercial additives currently on the market to fail in accelerating the establishment of nitrification in newly set-up aquaria because these additives do not contain the correct species of bacteria (further detailed in the following section).

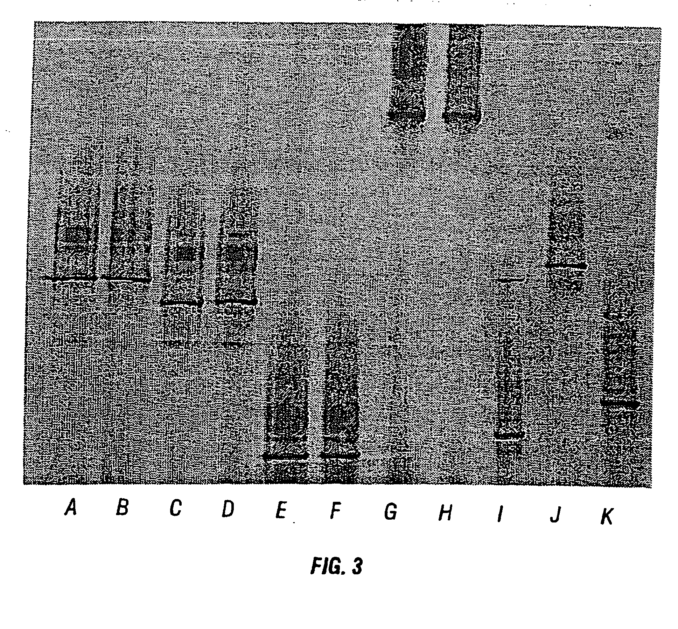

**Denaturing gradient gel electrophoresis (DGGE) survey of clones and reactors.** The novelty of the four bacteria strains reported herein are further demonstrated by the results of the DGGE testing. Figure 3 shows the DGGE results for 2 clone representatives for each of the Type A AOB (SEQ ID NO:1), Type A1 AOB (SEQ ID NO:2), Type B AOB (SEQ ID NO:3), and Type C AOB (SEQ ID NO:4) in a general eubacterial DGGE. The bacterial sequence of each AOB Type claimed herein denatures at a different position in the gel. This shows uniqueness and provides another way to distinguish each AOB type of the others and known AOB. None of the four bacterial sequences co-migrated with *Nitrosomonas europaea, Nitrosospira multiformis* and *Nitrosomonas cryotolerans* (Fig. 3).

DGGE analyses of biomass extracted from various reactors confirms the results of the PCR and FISH testing.

**Bacterial Additive Tests.** A series of experiments were conducted to determine the effectiveness of various bacterial mixtures containing the bacterial strains herein compared to 1) control aquaria which did not receive a mixture, 2) aquaria which were innoculated with bacterial mixtures manufactured by other companies for use in tropical fish aquaria, and 3) preserved or stored bacterial mixtures of the bacterial strains herein.

Effectiveness of a mixture is demonstrated by showing that the ammonia-oxidizing bacterial strains herein oxidize ammonia in aquaria and, further, that when combined with other bacterial strains, such as those for nitrite oxidation, accelerate the establishment of nitrification in aquaria. Establishment of nitrification can be measured in at least three different ways. The first is counting the number days it takes since setting-up a new aquarium for the ammonia and nitrite concentrations in the aquarium water to reach a near 0 mg/l concentration. In a newly set-up freshwater aquarium, it typically takes about 14 days for the ammonia concentration to reach 0 mg/l and about 30 to 35 days for nitrite to reach 0 mg/l. A second way to measure the beneficial action of adding nitrifying bacterial strains to aquaria is to compare the maximum concentration of ammonia or nitrite reached before the concentration drops to 0 mg/l. If the maximum concentration of ammonia or nitrite reached in aquaria in which nitrifying bacteria were added is significantly less than the maximum concentration reached in control aquaria then a degree of effectiveness is demonstrated. A third way to evaluate the effectiveness of nitrifying bacterial strains and mixtures that incorporate them is to combine the first two methods to form a toxicity exposure curve. This type of curve takes into account both the duration of the exposure (time in days) and the degree or intensity of the exposure. This curve to determined by calculate the area encompassed by plotting the concentration of the toxin over time. The curve area is determined for each treatment and toxin. The treatments of one test are then compared to each other and the control of the same test. The control surface area can be given an arbitrary value of 1 and the other surface area calculated as a ratio to the control. In this way, if the calculated value of a treatment is greater than 1 it is more effective than the control while a value less than 1 means the treatment was less effective than the control and maybe inhibited the establishment of nitrification.

The experiments reported here are a representative sampling of numerous tests done to demonstrate the effectiveness and utility of the bacterial strains herein and products which incorporate them.

**Bacterial Additive Test VI.** The goal of this test was to evaluate the ability of four bacterial mixtures, with bacterial strains herein, to accelerate the establishment of nitrification in freshwater aquaria, and compare that ability to control aquaria which did not receive a bacterial inoculation of any kind.

Material and Methods. Twenty-seven 10-gallon aquaria and twenty-seven Penguin 170B (Marineland Aquarium Products) hang-on-the-back style power filters were sterilized, thoroughly rinsed, and allowed to air dry. Each aquarium was then filled with 10 lbs of rinsed aquarium gravel (RMC Lonestar #3) and the filter installed. The aquaria then received 35 1 of city tap water which had been filtered through activated carbon. After turning the filters on, the water level on each aquaria was marked so all could be topped-off with deionized water (DI) to account for any water loss due to evaporation and sampling. The filters ran overnight prior to the addition of the bacterial additives and fish.

On day 0 of the test, the aquaria were topped off with DI water and a baseline water sample taken for analysis. Carbon cartridges (Marineland Aquarium Products, Part No. PA 0133) were rinsed with tap water and placed in each filter. New BioWheels^{®} (Marineland Aquarium Products, Part No. PR 1935B) were placed in each filter. After thirty minutes each tank was innoculated with its designated bacterial additive or if a control aquaria it was not dosed with a bacterial mixture.

Thirty minutes after the experimental bacterial additives were added a second set of water samples were taken for analysis. Then five rosy barbs (*Puntius conchonius*) and one giant danio (*Danio aequipinnatus*) were added to each tank. The fish were fed approximately 0.4 grams of tropical fish flake split into two feeding each day (approximately 9:00 a.m and and 4:30 p.m.).

Water samples were collected and analyzed tested daily for pH, ammonia, nitrite, and conductivity. Monday, Wednesday and Friday the water was tested for nitrate and turbidity. Anions and cations were measured periodically. Measurements for pH were made with a Denver Instruments Model 225 pH/Ion meter equipped with a Denver Instruments pH combination electrode. A Tecator FIAstar 5010 Analyzer was used to measure ammonia, nitrite, and nitrate (as nitrogen) using methods described in Tecator Application Notes. Cations (sodium, ammonium-nitrogen, potassium, magnesium, and calcium) were analyzed using a Dionex DX500 System with a CS15 4-mm Analytical Column. Specific conductance was measured directly in each tank at approximately 12:30 p.m. daily using a YSI Model 30 hand-held salinity, conductivity, and temperature system. Turbidity data was determined with a DRT-100 turbidity meter (HF Scientific).

Four bacterial mixtures used in this test. There were two dosing levels within each treatment: either 30 or 100 ml of a mixture per aquaria. There were three replicates of each mix/dose combination plus three control aquaria which did not receive a bacterial mixture for a total of 27 test aquaria ((4x2x3)+3=27). The bacterial mixtures and their conditions were: 1) BC5 - a bacterial mixture which had been under culture for 553 days preceding the test. A positive result with this mixture would demonstrate the long-term viability of the bacteria under culture conditions and the appropriateness of the culture techniques; 2) Rtr3 - this is a bacterial mixture which had been bottled and stored in the dark for 118 days preceding the test. A positive result with this mixture would demonstrate that the storage method is valid and the mixture retains its viability for at least 119 days of storage; 3) Rtr4 - this is a bacterial mixture which had been bottled and stored in the dark for 118 days preceding the test. A positive result with this mixture would demonstrate that the storage method is valid and the mixture retains its viability for at least 119 days of storage; 4) Rtr7 - this is a bacterial mixture which had been grown from an innoculum from BC5. A positive result with this mixture would demonstrate that one can culture the bacterial consortium in the mixture for successive generations and it maintains its viability.

Results and Discussion: This test was conducted for 23 days. At this time the ammonia and nitrite concentrations in all the aquaria were virtually 0 mg/L. However, there were significant differences in a) the mean highest ammonia and nitrite concentrations and b) the time, in days, it took to reach a zero mg/L concentration between all of the bacterial mixtures and the control aquaria. Further, there were some slight differences between the bacterial mixtures (Table 10).

**Table 9. Detection of Ammonia-oxidizing bacteria of the strains herein in various samples**

| **Template** | **Type A AOB PCR** | **Type B AOB PCR** | **Type C AOB PCR** | |
|---|---|---|---|---|
| R7c140 (Type) | +++ | - | - | |
| R7c187(TypeA) | +++ | - | - | |
| R3c5 (TypeB) | - | +++ | - | |
| RSc20 (TypeB) | - | +++ | - | |
| R3c12 (TypeC) | - | - | +++ | |
| RSc47 (TypeC) | - | - | +++ | |
| *N. europeae* | - | - | - | |
| *N. multiformis* | - | - | - | |
| *N. cryotolerans* | - | - | - | |
| BC5 Pre BA 6 | - | - | - | |
| BC5 Post BA 6 | + | - | - | |
| R3 Pre BA6 | + | + | +/- | |
| R3 Post BA 6 | + | +/- | - | |
| R4 Pre BA6 | + | + | - | |
| R4 Post BA 6 | ++ | - | - | |
| R5 Pre BA 7 | - | ++ | ++ | |
| R5 Post BA7 | - | - | - | |
| R7 Pre BA6 | ++ | + | - | |
| R7 Post BA 6 | ++ | - | - | |
| R7 Pre BA7 | + | +/- | - | |
| R7 Post BA7 | ++- | - | - | |
| Cycle | - | - | - | |
| Fritzyme | - | - | - | |
| Stresszyme | - | - | - | |
| Cryst Clr Nitrifier | - | - | - | |
| Cryst Clr Bio Clar L | - | - | - | |
| Cryst Clr Bio Clar S | - | - | - | |
| Acqmar Phospaht | - | - | - | |
| Trop Sci Sludge | - | - | - | |
| Trop Sci Rapid Act | - | - | - | |

| | | | | |
|---|---|---|---|---|
| +++ very strong presence, clearly indicates high amount of target organism ++ strong presence, indicates significant detection of signal + clear presence, signal detected =/- possible presence, signal weak but above background - no presence/signal detected | | | | |

**Table 10. Time to reach an ammonia or nitrite concentration of 0.50 mg/L-N or less for mixtures used in Bacterial Additives Test VI along with the mean maximum ammonia and nitrite concentrations reached during the test (N=3).**

| **Bacterial Mixture** | **Time to ≤0.50 mg/L (days)** | | **Maximum Concentration (mg/I-N)** | |
|---|---|---|---|---|
| | **Ammonia** | **Nitrite** | **Ammonia** | **Nitrite** |
| Rtr7 - 100 ml | 6 | 7 | 1.1 | 1.4 |
| Rtr3 - 100 ml | 7 | 11 | 1.9 | 3.4 |
| Rtr7 - 30 ml | 7 | 10 | 1.9 | 3.7 |
| BC5 - 100 ml | 8 | 18 | 2.4 | 4.5 |
| Rtr4 - 100 ml | 8 | 8 | 2.7 | 0.6 |
| Rtr3 - 30 ml | 9 | 15 | 3.1 | 5.9 |
| Rtr4 - 30 ml | 9 | 10 | 2.9 | 2.1 |
| BC5 - 30 ml | 10 | 21 | 4.1 | 8.9 |
| Control | 12 | 23 | 4.9 | 13.4 |

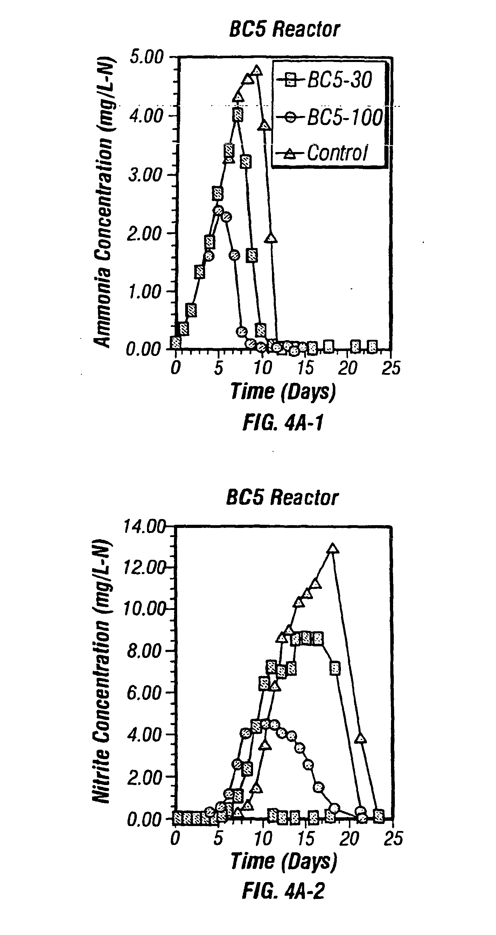

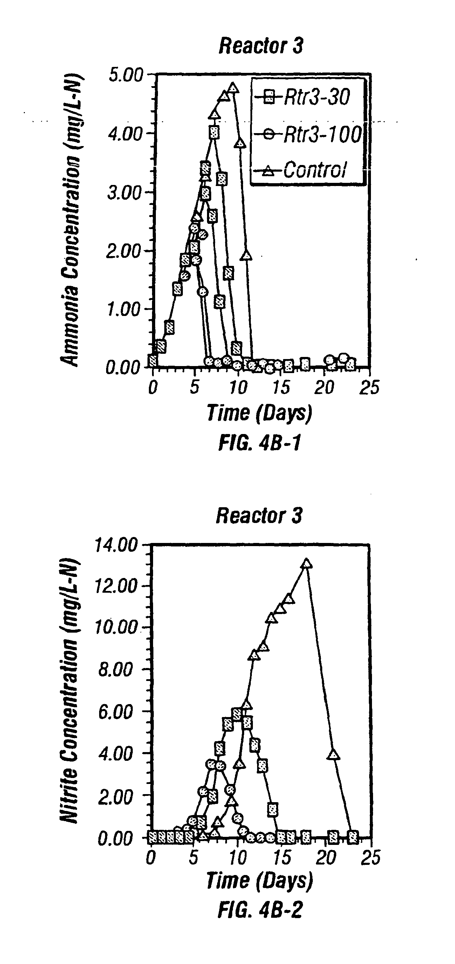

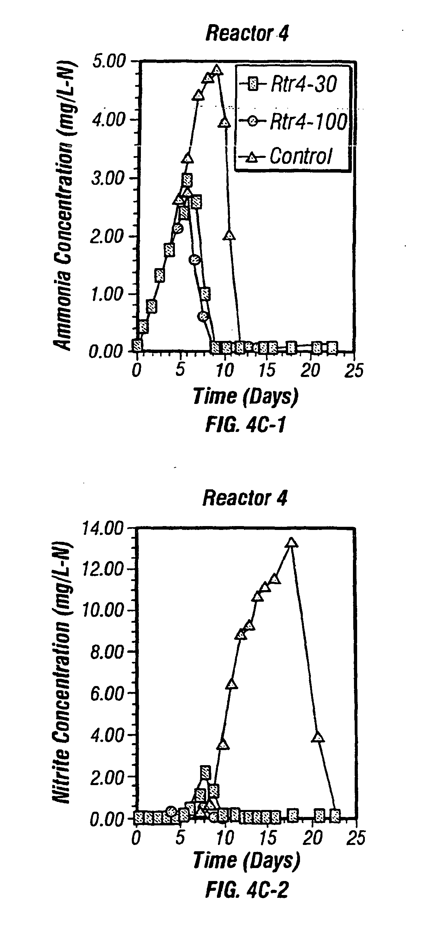

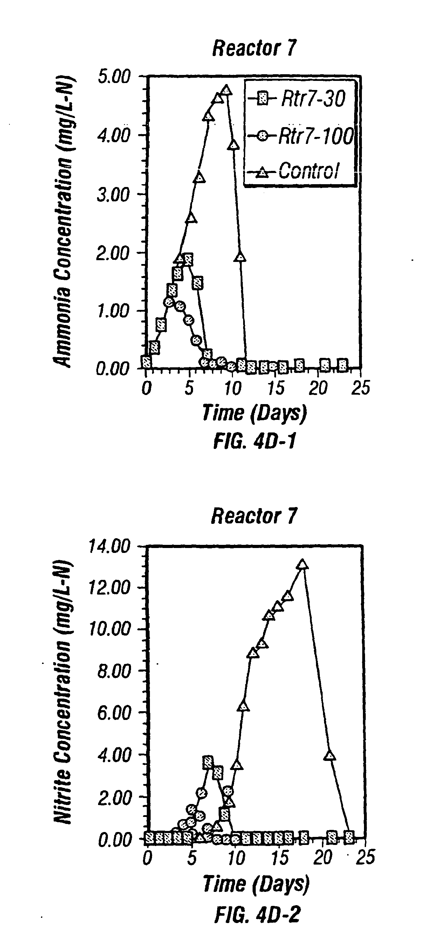

Figure 4 shows the mean ammonia and nitrite concentration over the test period for the four mixtures along with the controls. For the BC5 mixture, ammonia reached 0 mg/l on day 8 for the aquaria dosed with 100 ml of BC5 mixture and day 10 for the aquaria dosed 30 ml of BC5 mixture. The ammonia concentration in the control aquaria did not reach 0 mg/l until day 12. More importantly, the highest mean ammonia concentration reached for the control aquaria was 4.9 mg/L NH₃-N. However, for the aquaria dosed 30 ml of BC5 bacterial mixture the mean highest ammonia concentrations was 4.1 mg/l NH₃-N and for the aquaria dosed 100 ml of the BC5 bacterial mixture the mean highest ammonia concentrations was 2.4 mg/l NH₃-N. Thus, the addition of the BC5 bacterial mixture to newly set-up aquaria resulted in less ammonia exposure to the fish.

The ammonia exposure area curve values for the aquaria dosed with 30 or 100 ml of the BC5 mixture were 67% and 37% of the control aquaria curve area value, respectively (Table 11). These values show that the addition of the mixture resulted in 1.5 and 2.7 times less exposure to ammonia, respectively, to the fish in the treatment aquaria compared to the control aquaria.

**Table 11. Toxicity exposure data for the Bacterial Additives VI test.**

| **Ammonia** | | | | **Nitrite** | | | |
|---|---|---|---|---|---|---|---|
| **Treatment** | **Exposur e Value** | **% of control** | **Area** | **Treatment** | **Exposur** e **Value** | **% of control** | **Area** |
| **Rtr7-100** | 5.7 | 17% | 5.34 | **Rtr4-100** | 60.9 | 2% | 1.99 |
| **Rtr33-100** | 3.9 | 26% | 7.87 | **Rtr7-100** | 25.5 | 4% | 4.75 |
| **Rtr7-30** | 3.6 | 28% | 8.59 | **Rtr4-30** | 20.2 | 5% | 6.01 |
| **BC5-100** | 2.7 | 37% | 11.34 | **Rtr7-30** | 9.7 | 10% | 12.45 |
| **Rtr4-100** | 2.7 | 38% | 11.47 | **Rtr3-100** | 8.8 | 11% | 13.81 |
| **Rtr4-30** | 2.2 | 45% | 13.70 | **Rtr3-30** | 3.6 | 28% | 33.73 |
| **Rtr3-30** | 2.2 | 45% | 13.71 | **BC5-100** | 3.1 | 33% | 39.37 |
| **BC5-30** | 1.5 | 67% | 20.38 | **BC5-30** | 1.3 | 75% | 90.56 |
| **Control** | 1.0 | 100% | 30.56 | **Control** | 1.0 | 100% | 121.13 |

In terms of the nitrite concentrations for aquaria dosed with the BC5 bacterial mixture, the aquaria which received 100 ml of BC5 mixture reached 0 mg/l of nitrite by day 18, those dosed 30 ml of BC5 mixture reached 0 mg/l NO₂-N on day 21. The control aquaria reached 0 mg/l on day 23. Of even greater significance were the results for the maximum nitrite concentrations. The control aquaria reached a mean maximum nitrite control of 13.4 mg/L NO₂-N. The aquaria dosed with 30 ml of the BC5 mixture had a maximum mean nitrite concentration of 8.9 mg/l NO₂-N and the aquaria dosed with 100 ml of BC5 mixture had a maximum nitrite concentration of only 4.5 mg/l NO₂-N (Table 10; Fig. 4).

The nitrite exposure area curve values for the aquaria dosed with 30 or 100 ml of the BC5 mixture were 75% and 33% of the control aquaria curve area value, respectively (Table 11). These values show that the addition of the mixture resulted in 1.3 and 3.1 times less exposure to nitrite, respectively, to the fish in the treatment aquaria compared to the control aquaria.

The bacterial mixture Rtr3, which had been stored for 118 days, demonstrated a significantly faster time to establish nitrification compared to the control aquaria. The mean maximum ammonia concentration for the aquaria dosed 30 or 100 ml of the Rtr3 mixture was 3.1 NH₃-N and 1.9 mg/l NH₃-N, respectively (Table 10). This is in contrast to the control aquaria which had a mean maximum ammonia concentration of 4.9 mg/l NH₃-N. It took the control aquaria 12 days to reach a 0 mg/l ammonia concentration while the aquaria dosed 30 or 100 ml of the Rtr3 bacterial mixture took only 9 and 7 days to reach 0 mg/l NH₃-N, respectively (Table 10).

The nitrite concentration reached a mean maximum concentration of 13.4 mg/l NO₂-N for the control aquaria while for the aquaria dosed 30 or 100 ml of the Rtr3 bacterial mixture the mean maximum nitrite concentration was only 5.9 NO₂-N and 3.4 mg/l NO₂-N, respectively. The control aquaria reached a 0 mg/l nitrite concentration in 23 days, while it took only 15 and 11 days, respectively, for the aquaria dosed 30 or 100 ml of Rtr3 bacterial mixture to reach 0 mg/l NO₂-N (Table 10).

The ammonia exposure area curve values for the aquaria dosed with 30 or 100 ml of the Rtr3 mixture were 45% and 26% of the control aquaria curve area value, respectively (Table 11). These values show that the addition of the mixture resulted in 2.2 and 3.9 times less exposure to ammonia, respectively, to the fish in the treatment aquaria compared to the control aquaria.

The nitrite exposure area curve values for the aquaria dosed with 30 or 100 ml of the Rtr3 mixture were 28% and 11% of the control aquaria curve area value, respectively (Table 11). These values show that the addition of the mixture resulted in 3.6 and 8.8 times less exposure to nitrite, respectively, to the fish in the treatment aquaria compared to the control aquaria.

For the aquaria dosed with the Rtr4 mixture, the mean maximum ammonia concentration was 2.9 NH₃-N and 2.7 mg/L NH₃-N, respectively, for a dosage volume of 30 and 100 ml (Table 10). The control aquaria reached a mean maximum ammonia concentration of 4.9 mg/l NH₃-N. The ammonia exposure area curve values for the aquaria dosed with 30 or 100 ml of the Rtr4 mixture were 45% and 38% of the control aquaria curve area value, respectively. These values show that the addition of the mixture resulted in 2.2 and 2.7 times less exposure to ammonia, respectively, to the fish in the treatment aquaria compared to the control aquaria (Table 11). Aquaria dosed 30 ml of the Rtr4 mixture completed the nitrification cycle in 10 days. Nitrification was established in 8 days for aquaria dosed 100 ml of the Rtr4 (Table 10). These times are compared to 23 days for establishment of nitrification in the control aquaria.

The mean maximum nitrite concentration for the aquaria dosed 30 or 100 ml of the Rtr4 bacterial mixture was 2.1 NO₂-N and 0.6 mg/l NO₂-N respectively. The control aquaria had a mean maximum nitrite concentration of 13.4 mg/l NO₂-N.

The nitrite exposure area curve values for the aquaria dosed with 30 or 100 ml of the Rtr4 mixture were 5% and 2% of the control aquaria curve area value, respectively. These values show that the addition of the mixture resulted in 20.2 and 60.9 times less exposure to nitrite, respectively, to the fish in the treatment aquaria compared to the control aquaria (Fig. 4; Table 11).

The bacterial mixture Rtr7, which was a subculture from the BC5 mixture, demonstrated a significantly faster time to establish nitrification compared to the control aquaria. It took the control aquaria 12 days to reach a 0 mg/l NH₃-N ammonia concentration while the aquaria dosed 30 or 100 ml of the Rtr7 bacterial mixture took only 7 and 6 days to reach 0 mg/l NH₃-N, respectively (Table 10). The mean maximum ammonia concentration for the aquaria dosed 30 or 100 ml of the R7 mixture was 1.9 NH₃-N and 1.1 mg/l NH₃-N, respectively. This is in contrast to the control aquaria which had a mean maximum ammonia concentration of 4.9 mg/l NH₃-N (Table 10).

The nitrite concentration reached a mean maximum concentration of 13.4 mg/l NH₃-N for the control aquaria while for the aquaria dosed 30 or 100 ml of the Rtr7 bacterial mixture the mean maximum nitrite concentration was only 3.7 NO₂-N and 1.4 mg/l NO₂-N respectively (Table 10). The control aquaria reached a 0 mg/l NO₂-N nitrite concentration in 23 days, while it took only 10 and 7 days, respectively, for the aquaria dosed 30 or 100 ml of Rtr7 bacterial mixture to reach 0 mg/l NO₂-N (Table 10; Fig. 4)

The ammonia exposure area curve values for the aquaria dosed with 30 or 100 ml of the Rtr7 mixture were 28% and 17% of the control aquaria curve area value, respectively (Table 11). These values show that the addition of the mixture resulted in 3.6 and 5.7 times less exposure to ammonia, respectively, to the fish in the treatment aquaria compared to the control aquaria.

The nitrite exposure area curve values for the aquaria dosed with 30 or 100 ml of the Rtr7 mixture were 10% and 4% of the control aquaria curve area value, respectively. These values show that the addition of the mixture resulted in 5.7 and 25.5 times less exposure to nitrite, respectively, to the fish in the treatment aquaria compared to the control aquaria (Table 11).

In summary, the data from the test show that the various bacterial mixtures, which contain bacterial strains incorporated herein, are effective at accelerating the establishment of nitrification in aquaria. Use of the mixtures, incorporating the bacterial strains herein, in aquaria significantly reduced the degree of ammonia and nitrite exposure to the fish in the innoculated aquaria versus the fish in control aquaria. Specifically, the results demonstrate that a mixture can be viably maintained over a long period of time (e.g., BC5), that the mixture can be stored for several months (e.g., Rtr 3 and Rtr 4), and that successive generations of the mixture retain the ability to nitrifying (e.g., Rtr 7).

**Bacterial Additive Test VII.** The goal of this test was to evaluate two mixtures of the bacterial stains reported herein as they would be used in a 'real world' setting and, at the same time, compare their performance to that of bacterial mixtures currently sold in the aquarium industry by other companies.

In general, a new aquarium owner first purchases all the necessary equipment for setting-up an aquarium capable of maintain aquatic life. The equipment includes the aquarium, decorations, a heater and filter, and a water conditioner. The aquarium is then set-up and filled with water, the filters started, the heater adjusted to the proper water temperature and the water conditioner added to remove chlorine. At this point the fish are usually added but there are insufficient populations of ammonia- and nitrite-oxidizing bacteria to maintain the ammonia and nitrite concentrations in the aquarium at safe concentrations (below 0.5 mg/l-N). Therefore, the newly set-up aquarium will experience what is called "new tank syndrome". New tank syndrome is the evaluated concentrations of ammonia and nitrite that occur in the first several weeks of setting-up a new aquarium when there are insufficient number of nitrifying bacteria to maintain ammonia and nitrite concentrations at safe levels.

Thus, many times a new aquarium owner will purchase a bottled mixture of microorganisms or an enzyme mix (the bacterial mixture) which state on the label that they accelerate, or in some cases, eliminate new tank syndrome. What this means is that the use of the bottled mixture should result in significantly lower ammonia and nitrite concentrations in the aquarium during the initial several weeks then if one were not to use a mixture. Furthermore, the length of time before the ammonia and nitrite concentrations in the aquarium reach 0 mg/l should be less with using a bacterial mixture.

Materials and Methods. Thirty-three 10-gallon aquaria and thirty-three Penguin 170B (Marineland Aquarium Products) hang-on-the-back style power filters were sterilized, thoroughly rinsed, and allowed to air dry. Then each aquarium was filled with 10 lbs of rinsed aquarium gravel (RMC Lonestar #3) and the filter set-up on the back. Next, each aquaria was filled with 35L of city water, which had been prefiltered through activated carbon, and the water level marked on each aquaria. This mark was used as a guide for when the aquaria water needed to be topped-off to make up for water lost due to evaporation or sampling. Deionized water was used to top-off the aquaria. The filters were allowed to run overnight prior to the addition of bacterial additives and fish.

On the first day of the test, the aquaria were topped off with deionized water to account for water and a baseline water sample. Carbon cartridges (Marineland Aquarium Products, Part No. PA 0133) were rinsed with tap water and placed in each filter. New BioWheels^{®} (Marineland Aquarium Products, Part No. PR 1935B) were placed in each filter. After thirty minutes each tank was dosed with its designated bacterial additive. The dosages were as given in Table 12. Thirty minutes after the bacterial additives were added a second set of water samples were taken for analysis. Then six assorted barbs [(*Puntius conchonius*) Rosy Barbs; *(Puntius tetrazona*) Albino Tiger Barbs, and Tiger Barbs] were added to each tank. The fish in each aquarium were fed twice a day (at about 9:00 a.m. and again at 4:30 p.m.) for a total of 0.4 grams of tropical fish flakes per day.

Water samples were collected and analyzed tested daily for pH, ammonia, nitrite, and conductivity. Monday, Wednesday and Friday the water was tested for nitrate and turbidity. Anions and cations were measured periodically. Measurements for pH were made with a Denver Instruments Model 225 pH/Ion meter equipped with a Denver Instruments pH combination electrode. A Tecator FIAstar 5010 Analyzer was used to measure ammonia, nitrite, and nitrate (as nitrogen) using methods described in Tecator Application Notes. Cations (sodium, ammonium-nitrogen, potassium, magnesium, and calcium) were analyzed using a Dionex DX500 System with a CS15 4-mm Analytical Column. Specific conductance was measured directly in each tank at approximately 12:30 p.m. daily using a YSI Model 30 hand-held salinity, conductivity, and temperature system. Turbidity data was determined with a DRT-100 turbidity meter (HF Scientific).

For this test two formulations containing bacterial strains reported herein were tested along with four commercially available bacterial mixtures. On the first day of the test 100 ml of the first formulation (called Rtr5) was added to each of four aquaria, and 100 ml of the second formulation (called Rtr7) was added to another four aquaria.

The commercially available bacterial mixtures were dosed according to the manufacturers instructions, for the treatments of Biozyme®, Cycle®, Fritz-Zyme No.7® and Stress Zyme®. Furthermore, each of the above commercially available bacterial mixtures was also tested at three times the recommended dosing level (Table 12). There were four replicate aquaria per treatment/dosage combination for a total of 33 aquaria ((((4x3)x2)+(2X3)+3)=33).

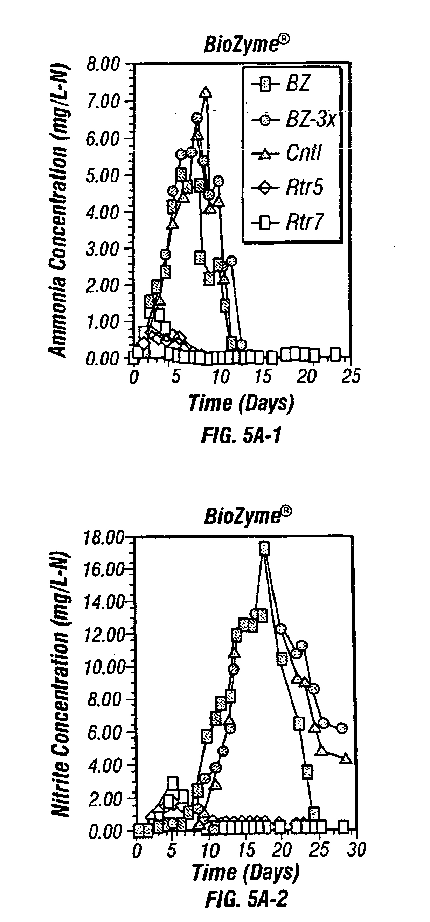

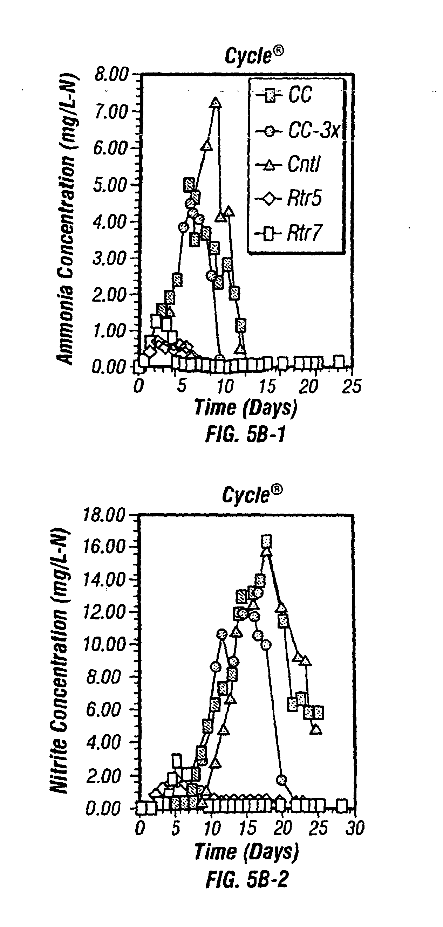

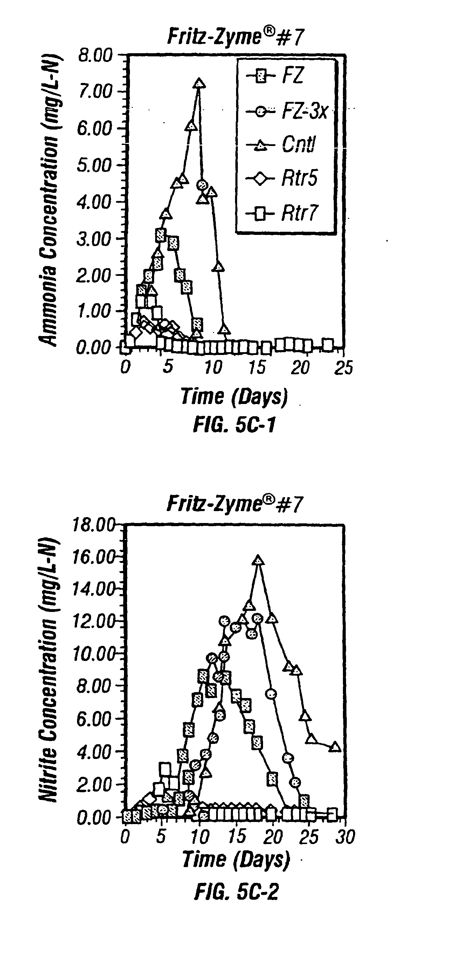

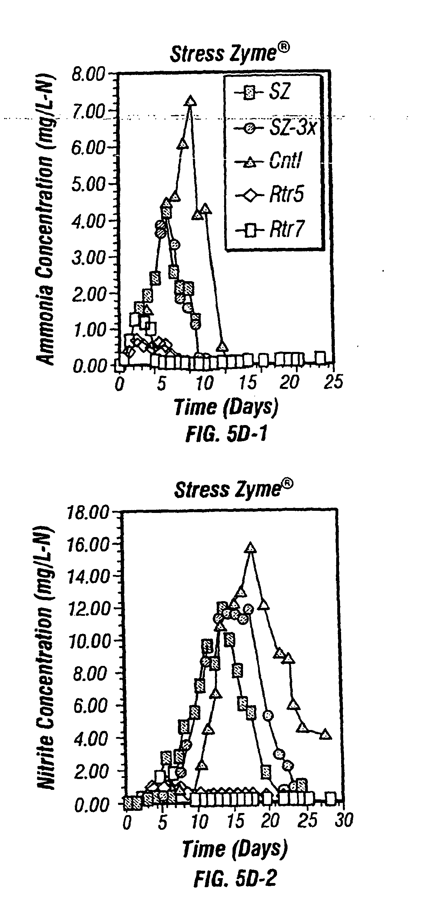

Results and Discussion. The ammonia and nitrite trends for the treatments and control for Bacterial Additives Test VII are shown in Figure 5. For clarity of presentation, each of the commercially available bacterial mixtures tested is presented with the control and the two test mixtures containing the bacterial strain herein. The scale of each plot is the same so comparisons between all the treatments can be easily made. The data show that the Rtr5 and Rtr7 mixtures, containing the bacterial stains herein, significantly decreased the time to establish nitrification in newly set-up aquaria compared to aquaria which were not dosed (the controls) or which received a commercially available bacterial mixture. Furthermore, the maximum ammonia and nitrite concentrations reached in the aquaria which were dosed with either the Rtr5 and Rtr7 mixtures were significantly lower than all the other treatments (Fig. 5; Table 13).

**Table 13. Time to reach an ammonia or nitrite concentration of 0.50 mg/L-N or less for mixtures used in Bacterial Additives Test VII along with the mean maximum ammonia and nitrite concentrations reached during the test.**

| | **Time to ≤0.50 mg/L (days)** | | **Maximum Concentration (mg/l-N)** | |
|---|---|---|---|---|
| **Bacterial Mixture** | **Ammonia** | **Nitrite** | **Ammonia** | **Nitrite** |
| Rtr7 | 6 | 8 | 2.8 | 1.3 |
| Rtr5 | 8 | 10 | 1.5 | 0.9 |
| Fritz-Zyme®3x | 10 | 24 | 8.5 | 3.1 |
| Fritz-Zyme" | 11 | 22 | 7.2 | 3.1 |
| Cycle®3x | 11 | 22 | 8.4 | 4.5 |
| Stress Zyme® | 12 | 22 | 8.3 | 4.1 |
| Stress Zyme®3x | 12 | 25 | 8.6 | 4.3 |
| Biozyme® | 14 | 25 | 8.6 | 5.1 |
| Biozyme®3x | 15 | 30+ | 8.8 | 6.5 |
| Cycle® | 15 | 30 | 8.8 | 4.3 |
| Control | 15 | 30 | 8.9 | 7.2 |

The results show that the Rtr5 and Rtr7 mixtures were capable of establishing nitrification in newly set-up aquaria much faster than currently available commercial mixtures and aquaria not dosed with any mixture. Complete nitrification was established in 8 days with the Rtr7 mixture and 10 days with the Rtr5 mixture (Table 13). The closest treatments to these were the Fritz-Zyme® at the normal dosing level, Cycle® at three times the normal dosing level, and Stress Zyme® at the normal dosage level all of which took 22 days (Table 13). The Rtr5 and Rtr7 mixtures were 2.2 to 2.8 times faster at establishing nitrification then these other mixtures.

The difference in the maximum concentration of ammonia or nitrite reached for the various mixtures and control were also significantly different (Table 13). The mean (N=3) maximum ammonia concentration of 1.5 mg/L NH₃-N reached during the test for the Rtr5 mixture was 4.8 times less than the Fritz-Zyme® (mean 7.2 mg/L NH3-N, N=3), dosed at the normal level, which was the nearest commercially available mixture (Table 13). The mean maximum nitrite concentration for the Rtr5 mixture was 0.9 mg/L NO2-N. Again, Fritz-Zyme® dosed at the normal level was the closest commercially available mixture with a mean maximum nitrite concentration of 3.1 mg/L NO2-N. Thus the Rtr5 mixture was 3.4 times more effective at establishing nitrification then the presently available commercial mixtures tested. The Rtr7 mixture exhibited the same trend as the Rtr5 mixture in that aquaria which were dosed with this mixture had significantly lower maximum ammonia-nitrogen and nitrite-nitrogen concentrations than the aquaria dosed with the commercially available bacterial mixtures (Table 13). The Rtr7 mixture had mean maximum ammonia and nitrite concentrations of 2.8 and 1.3 mg/L, respectively. These were 2.6 and 2.4 times lower, respectively, than the closest commercially available bacterial mixture (Fritz-Zyme® dosed at the normal recommended level) (Figure 5; Table 13).

In terms of the exposure curves, the bacterial mixtures Rtr5 and Rtr7, which incorporate the bacterial strains herein, significantly outperformed the commercially available mixtures tested (Table 14). Rtr7 performed the best of all the mixtures tested with the fish exposed to just 13% of the ammonia and 5% of the nitrite as compared to the control. Rtr5 was almost as good with ammonia exposure 14% of the control and nitrite exposure 9% of the control (Table 14). These results mean that fish in an aquarium receiving either Rtr7 or Rtr5 were exposed to 7.3 to 7.6 less ammonia and 11.6 and 19.6 times less nitrite than fish in the control aquaria. The next best mixtures only reduced the exposure of ammonia and nitrite by 50% compared to the controls (Table 14).

**Table 14. Toxicity exposure data for the Bacterial Additives VII test.**

| **Ammonia** | | | | | **Nitrite** | | | |
|---|---|---|---|---|---|---|---|---|
| **Treatment** | **Exposure Value** | **% of control** | **Area** | | **Treatment** | **Exposure Value** | **% of control** | **Area** |
| **Rtr7** | 7.6 | 13% | 5.90 | | **Rtr7** | 19.6 | 5% | 8.16 |
| **Rtr5** | 7.3 | 14% | 6.11 | | **Rtr5** | 11.6 | 9% | 13.85 |
| **Fritz 3x** | 2.6 | 39% | 17.52 | | **Fritz 1x** | 1.9 | 52% | 82.71 |
| **Fritx 1x** | 2.5 | 40% | 17.84 | | **Stress 1x** | 1.8 | 55% | 88.08 |
| **Stress 3x** | 1.9 | 52% | 23.50 | | **Cycle 3x** | 1.6 | 63% | 100.05 |
| **Stress 1x** | 1.9 | 53% | 23.76 | | **Stress 3x** | 1.3 | 75% | 119.42 |
| **Cycle 3x** | 1.7 | 59% | 26.36 | | **Fritz 3x** | 1.2 | 84% | 134.60 |
| **Cycle 1x** | 1.3 | 76% | 34.03 | | **Bio 1x** | 1.1 | 88% | 141.50 |
| **Bio 1x** | 1.3 | 79% | 35.29 | | **Control** | 1.0 | 100% | 159.97 |
| **Control** | 1.0 | 100% | 44.82 | | **Cycle 1x** | 1.0 | 104% | 166.83 |
| **Bio 3x** | 0.9 | 111% | 49.90 | | **Bio 3x** | 0.9 | 114% | 182.30 |

## Claims

1. An isolated bacterial strain that oxidizes ammonia to nitrite, said strain comprising the sequence set forth in SEQ ID No: 3.

2. A biologically pure culture of a bacterial strain that oxidizes ammonia to nitrite, wherein the 16S rDNA of the bacterial strain comprises the sequence set forth in SEQ ID No: 3.

3. A composition comprising an isolated bacterial strain that oxidizes ammonia to nitrite, wherein said bacterial strain comprises the sequence set forth in SEQ ID No: 3

4. The composition according to claim 2 or 3 which is in a frozen form.

5. The composition according to claim 2 or 3 which is in a freeze-dried form.

6. The composition according to claim 2 or 3, which is in the form of powder.

7. The composition according to any one of claims 2-6, which additionally comprises nitrite-oxidizing microorganisms, in a mineral salt solution.

8. The composition according to any one of claims 2-6, which additionally comprises nitrate-reducing microorganisms, in a mineral salt solution.

9. The composition according to any one of claims 2-6, which additionally comprises heterotrophic microorganisms in a mineral salt and organic based solution.

10. An isolated nucleotide sequence comprising the sequence set forth in SEQ ID No: 3

11. A method of alleviating or preventing the accumulation of ammonia in an aquarium comprising a step of placing into the aquarium a sufficient amount of a bacterial strain capable of oxidizing ammonia to nitrite to alleviate the accumulation of ammonia in the aquarium, wherein said bacterial strain comprises the sequence set forth in SEQ ID No: 3. or a variant thereof which is at least 99% similar.

12. The method according to claim 11, wherein ammonia is reduced by at least 30% over ammonia levels in an untreated aquarium.

13. The method according to claim 11 or 12, wherein the aquarium is a freshwater aquarium.

14. The method according to claim 11 or 12, wherein the aquarium is a seawater aquarium.

15. The method according to any one of claim 11 - 14, wherein the bacterial strain is placed into the aquarium by means of a rotating biological contactor.

16. The method according to any one off claim 11 - 14, wherein the bacterial strain is placed into the aquarium by means of a biofilter.

## Patentansprüche

1. Isolierter Bakterienstamm, der Ammoniak zu Nitrit oxidiert, wobei der Stamm die in SEQ ID NO: 3 dargelegte Sequenz umfasst.

2. Biologische Reinkultur eines Bakterienstammes, der Ammoniak zu Nitrit oxidiert, wobei die 16S-rDNA des Bakterienstammes die in SEQ ID NO: 3 dargelegte Sequenz umfasst.

3. Zusammensetzung, umfassend einen isolierten Bakterienstamm, der Ammoniak zu Nitrit oxidiert, wobei der Stamm die in SEQ ID NO: 3 dargelegte Sequenz umfasst.

4. Zusammensetzung nach Anspruch 2 oder 3, die in eingefrorener Form vorliegt.

5. Zusammensetzung nach Anspruch 2 oder 3, die in gefriergetrockneter Form vorliegt.

6. Zusammensetzung nach Anspruch 2 oder 3, die in der Form eines Pulvers vorliegt.

7. Zusammensetzung nach einem der Ansprüche 2 - 6, die zusätzlich Nitrit-oxidierende Mikroorganismen umfasst, in einer Mineralsalzlösung.

8. Zusammensetzung nach einem der Ansprüche 2 - 6, die zusätzlich Nitrat-reduzierende Mikroorganismen umfasst, in einer Mineralsalzlösung.

9. Zusammensetzung nach einem der Ansprüche 2 - 6, die zusätzlich heterotrophe Mikroorganismen umfasst, in einer Lösung auf Mineralsalz- oder organischer Basis.

10. Isolierte Nukleotidsequenz, umfassend die in SEQ ID NO: 3 dargelegte Sequenz.

11. Verfahren zur Verminderung oder Verhinderung der Akkumulation von Ammoniak in einem Aquarium, umfassend einen Schritt des Platzierens in das Aquarium einer ausreichenden Menge eines Bakterienstammes, der zum Oxidieren von Ammoniak zu Nitrit zur Verminderung der Akkumulation von Ammoniak im Aquarium fähig ist, wobei der Bakterienstamm die in SEQ ID NO: 3 dargelegte Sequenz oder eine Variante davon, die zu mindestens 99% ähnlich ist, umfasst.

12. Verfahren nach Anspruch 11, worin Ammoniak im Vergleich zu den Ammoniakspiegeln in einem unbchandelten Aquarium um mindestens 30% reduziert ist.

13. Verfahren nach Anspruch 11 oder 12, wobei das Aquarium ein Süßwasseraquarium ist.

14. Verfahren nach Anspruch 11 oder 12, wobei das Aquarium ein Meerwasseraquarium ist.

15. Verfahren nach einem der Ansprüche 11 - 14, wobei der Bakterienstamm mittels eines Scheibentauchtropfkörpers in das Aquarium platziert wird.

16. Verfahren nach einem der Ansprüche 11 - 14, wobei der Bakterienstamm mittels eines Biofilters in das Aquarium platziert wird.

## Revendications

1. Souche bactérienne isolée qui oxyde l'ammoniac en nitrite, ladite souche comprenant la séquence décrite dans la SEQ ID No: 3.

2. Culture biologiquement pure d'une souche bactérienne qui oxyde l'ammoniac en nitrite, dans laquelle le 165 ADNr de la souche bactérienne comprend la séquence décrite dans 1a SEQ ID No: 3.

3. Composition comprenant une souche bactérienne isolée qui oxyde l'ammoniac en nitrite, dans laquelle ladite souche bactérienne comprend la séquence décrite dans la SEQ ID No: 3.

4. La composition selon la revendication 2 ou 3, qui est sous forme congelée.

5. La composition selon la revendication 2 ou 3, qui est sous forme lyophilisée.

6. La composition selon la revendication 2 ou 3, qui est sous forme de poudre.

7. La composition selon l'une quelconque des revendications 2 à 6, qui comprend en plus des microorganismes oxydant le nitrite dans une solution de sel minéral.

8. La composition selon l'une quelconque des revendications 2 à 6, qui comprend en plus des microorganismes réducteurs de nitrate dans une solution de sel minéral.

9. La composition selon l'une quelconque des revendications 2 à 6, qui comprend en plus des microorganismes hétérotrophes dans une solution de sel minéral et à base organique.

10. Séquence de nucléotides isolée comprenant la séquence présentée dans la SEQ ID No: 3.

11. Méthode pour alléger ou empêcher l'accumulation d'ammoniac dans un aquarium, comprenant l'étape consistant à mettre dans un aquarium, une quantité suffisante d'une souche bactérienne capable d'oxyder l'ammoniac en nitrite pour alléger l'accumulation d'ammoniac dans l'aquarium, où ladite souche bactérienne comprend la séquence présentée dans la SEQ ID No: 3 ou une variante de celle-ci, qui est au moins semblable à 99%.

12. La méthode selon la revendication 11, dans laquelle l'ammoniac est réduit d'au moins 30% par rapport aux taux d'ammoniac dans un aquarium non traité.

13. La méthode selon la revendication 11 ou 12, dans laquelle l'aquarium est un aquarium d'eau douce.

14. La méthode selon la revendication 11 ou 12, dans laquelle l'aquarium est un aquarium d'eau de mer.

15. La méthode selon l'une quelconque des revendications 11 à 14, dans laquelle la souche bactérienne est mise dans l'aquarium à l'aide d'un contacteur biologique rotatif.

16. La méthode selon l'une quelconque des revendications 11 à 14, dans laquelle la souche bactérienne est mise dans l'aquarium à l'aide d'un biofiltre.
